# EUROPEAN PATENT SPECIFICATION

(11) **EP 3 095 393 B1**
(45) Date of publication and mention of the grant of the patent: **12.12.2018**
(21) Application number: 15168542.7
(22) Date of filing: 20.05.2015
(51) Int. Cl.: A61B 17/064, A61B 17/068, A61B 17/00

(54) **SURGICAL STAPLE AND INSTRUMENT FOR HOLDING AND IMPLANTING THE SURGICAL STAPLE**
CHIRURGISCHE KLAMMER UND INSTRUMENT ZUM HALTEN UND IMPLANTIEREN DER CHIRURGISCHEN KLAMMER
AGRAFE CHIRURGICALE ET INSTRUMENT PERMETTANT DE TENIR ET D'IMPLANTER UNE TELLE AGRAFE

(43) Date of publication of application: 23.11.2016
(73) Proprietor: Biedermann Technologies GmbH & Co. KG, 78166 Donaueschingen (DE)
(72) Inventor: Biedermann, Lutz, 78048 VS-Villingen (DE); Biedermann, Timo, 78647 Trossingen (DE); Zipse, Achim, 76532 Baden-Baden (DE); Dreher, Gael, 76227 Karlsruhe (DE)
(74) Representative: Prüfer & Partner mbB Patentanwälte · Rechtsanwälte

(56) References cited:
- WO-A1-2010/004602
- WO-A1-2016/154417
- DE-A1- 4 110 123
- DE-C1- 19 821 680
- FR-A1- 2 725 126
- FR-A1- 2 999 069
- US-A- 4 263 903
- US-A- 5 454 814

## Description

The present invention relates to a surgical staple as used for compressing bones or bone fragments. The invention particularly relates to a surgical staple including a first leg for anchoring in a first bone or bone fragment, at least a second leg for anchoring in a second bone or bone fragment, and a bridge connecting the first leg and the second leg. The invention also relates to an instrument for holding and implanting the surgical staple.

Surgical staples are used to treat angular deformations, fractures particularly with respect to the extremities, subluxation, dislocation, arthritis etc. that may occur with regard to bones of the human body. The staples thereby tied to respective bones or bone fragments to fuse these together while exerting a compressive force on these parts. Staples are advantageous over other fusion techniques such as plates in view of the compactness and flexibility.

Document US 6,908,467 B2 discloses a distraction device made from a nitinol wire which includes an S-curve and bent end regions provided with hooks for anchoring in the bony material. When the distraction device is implanted, a rise of the temperature above the transfer temperature causes the shape memory material to change from a martensite state to an austenite state. The device is distracted when the S-curve assumes an elongated shape in the austenite state. The hooks self-lock in the opposing bone surfaces and the bone sections distract. Due to the superelastic characteristics of Nitinol, the distraction force remains more constant.

Document US 7,618,441 B2 discloses a bone staple which may be made of Nitinol. The staple has a bridge and each one leg extending from corner joints adjacent respective end portions of the bridge. The bridge may be slightly arc-shaped whereas the legs extend parallel to each other. The bridge also slightly extends beyond the corner joints thereby forming stop means or shoulders. The shoulders serve for reinforcing the connection between the legs and the bridge, and, since the level of the shoulder is lower than the bridge, for preventing the bridges from entering into contact with the bone. The staple is used to address convexity or concaveness of the vertebral column, wherein the shape memory effect of Nitinol is employed to splay the legs of the staple inserted into respective vertebrae away from each other.

Document GB 2471648 B discloses a staple for bones. The staple may be made of a shape memory material such as NiTi and has a connector including a ring of curved portions allowed to be distracted and further including three or four legs arranged parallel to each other.

Document US 2013/0026206 A1 discloses a bone staple which has a bridge connecting two legs and which is made from Nitinol. The bridge has an S-shape in a plane perpendicular to that of the legs and the legs are inclined toward each other in a closed configuration. In an opened configuration, the legs become parallel.

Document US 2013/0231667 A1 discloses a surgical staple having a middle section or bridge and side sections or legs which cantilever from ends of the middle section at an angle close to 90°. In a relaxed configuration, the middle section is curved within the plane of the side sections and has an angle of curvature between 15° and 35°. The side sections extend towards each other in this configuration. Use of Nitinol superelastic material is also disclosed therein.

Document US 2014/0277516 A1 discloses a bone staple including a bridge and legs extending from ends of the bridge. The bridge has a straight shape in a relaxed as well as in a splayed configuration of the legs. To splay the legs, the staple is put on a storage block and two tips of respective pivoting inserter handles force the side legs into the splayed configuration at 90° relative to the bridge. The staple is made from a super elastic and shape memory material such as Nitinol ASTM 2063.

Document US 2014/0358187 A1 discloses a surgical staple and a staple insertion device. The staple may be made of Nitinol and has a bridge and legs extending from ends of the bridge. To achieve a tensioned configuration in which the legs are parallel to each other, the staple is loaded to jaws of the insertion device which may be rotated outwards by means of a spacer configured to separate the jaws, which adversely engage the legs of the staple.

Document DE 41 10 123 A1 discloses an elastic connecting clip with the features as defined in the preamble of claim 1. The connecting clip has two legs and a base which is curved. A tensioning instrument may hold the clip in an expanded state via holding elements which engage corresponding engagement elements formed as grooves at respective ends of the base.

Document WO 2016/154417 A1 discloses a staple comprising a bridge configured to be elastically bendable, a first and a second leg connected to the bridge by respective hinge regions configured to be elastically bendable. The hinge regions each comprise a hole configured to mate with an element of a delivery device. The first and second legs are angled toward one another when they are in an unstrained state, whereby, when the staple is mounted to a delivery device so that the holes mate with respective elements of the delivery device, and further, when the delivery device applies a force to the bridge of the staple so as to reconfigure the bridge of the staple, the first and second legs are pivoted away from one another toward a parallel disposition.

It is the object of the invention to provide a surgical staple and an insertion holder which improves the process of insertion of the staple into the bones.

This object is solved by a surgical staple according to claim 1, and an assembly according to claim 8. Further advantageous embodiments become apparent from the appended claims.

According to the invention, a surgical staple for compressing bones or bone fragments includes a first leg and a second leg for anchoring in respective bones or bone fragments. A bridge connects the first leg and the second leg. The bridge has a first end section and a second end section opposite the first end section. The legs extend from the respective end sections.

The bridge is provided to be arc-shaped, or curved in at least a portion thereof, in a relaxed state of the staple (a state without external forces acting on the staple). This allows to achieve a straight shape with having, e.g., a flat bottom surface when being transferred into an expanded, or opened, state of the staple by means of an instrument. In the expanded state of the staple, the legs are generally brought into a configuration being parallel to each other which is suitable for insertion into pre-drilled holes in the bones or bone fragments.

The bridge may acquire mechanical energy when the staple is expanded and the bridge is bent such as to splay the legs towards, e.g., a parallel configuration. In other words, the bridge does not only connect the legs but also provides for achieving the expanded state of the legs. Advantageously, when the expanded state is realized using the staple of the invention, the bridge attains a straight shape with its, e.g., flat bottom side section thereby closely following the surface of the bones or bone fragment involved. As a consequence, the staple may consume less space within the soft tissue adjacent the bones or below the skin.

In order to allow the staple and its bridge thus expanded or bent respectively to be attached to bones or bone fragments, the surgical staple according to the invention further comprises two engagement portions for engagement by an external instrument. The engagement portions allow engaging the bridge and maintaining the bridge itself in a bent (expanded) state, the bridge thereby having for example the straight shape. According to specific examples not forming part of the invention, the engagement portion may be for example a recess or a projection having a surface facing or at least inclined towards one or both of the first and second legs. In many examples not forming part of the invention, there are at least two engagement portions provided at the bridge, which allows engaging the bridge from two sides.

Examples comprising at least one engagement portion are also described therein which, however, do not form part of the present invention. The one or more engagement portions are preferably provided to extend at or adjacent the end section(s) of the bridge. This allows to exert a pulling force onto the end sections by means of an instrument with high torque via the engagement portions while exerting a pressing for example the top surface in a centre section of the bridge in an opposite direction with the consequence, that the bridge bends and the legs move away from each other into a parallel configuration.

In one example the at least one engagement portion may also be located above a plane defined by a bottom surface of the expanded and thus straightly shaped bridge and a direction perpendicular to the legs. Such plane may correspond to a bone surface, when the surgical staple is inserted therein. An advantage thereby arises in that any engagement structures of the external instrument may engage the at least one engagement portion at a height level above that plane such that the legs of the staple can be inserted into pre-drilled holes in the bones or bone fragments until the, e.g., flat bottom side section of the bridge abuts on the bone surface.

As a result, no parts of the external tool are sandwiched between the bridge and the bone upon attachment of the staple to the bones or bone fragments such that it is not necessary to remove the tool first and then to hammer the staple into the holes. Rather, the staple may be fully arranged in position in the bones or bone fragments using the external instrument, only. Hence, the number of steps of insertion is also reduced.

According to an embodiment, the surgical staple may at least partially be made from a shape memory material, in particular Nitinol. The surgical staple is in its first, relaxed state upon fabrication in the austenitic phase. For easy insertion, the surgical staple is deformed into a desired second shape having parallel legs and a straight bridge for example as explained above. Deformation may be accomplished by a transfer instrument or pliers, for example. The deformed surgical staple may be transferred from the transfer instrument or pliers to a staple holder. Another option is to use the same plier for deformation from the relaxed shape into the shape for insertion as well as for performing the insertion into the bone(s) itself.

When inserting the staple into bones of the human body etc., a so called super- or pseudoelasticity of the shape-memory alloy is utilized. This allows the staple to exert a compression onto the bones involved after insertion and release from the staple holder or plier. The (super-)elastic deformation of the staple from its relaxed shape to its shape for insertion involves a phase transformation from austenite to martensite in the highly loaded areas of the staple. These areas remain under a certain stress after insertion of the staple and enable the staple to exert a force onto the bones and to compress them.

In line with the surgical staple of the invention, there is provided a staple holder or instrument. The staple holder includes a face formed with a cavity shaped to receive the bridge of the surgical staple in an expanded state thereof, wherein the cavity is provided with two engagement structures complementary to and cooperating with the respective two engagement portions provided at the bridge of the staple. The cavity thereby provides at least some of the reverse surface features of the bridge including the engagement portions. As the cavity is adjacent to the face and opens thereto, the bridge may safely be received in the cavity with the legs and the bottom side section of the bridge being exposed to the outside and ready for insertion to the bones.

According to one further example not forming part of the invention, there is provided an expansion device. The device includes a transfer portion provided on a boss which allows displacing the surgical staple from a first portion of the boss where it is received in its relaxed state to a second portion where it is expanded. The expansion may relate to an elastic deformation or a plastic deformation. An accommodation space which accommodates the staple holder is then structured such as to allow the bridge when reaching the second portion to be received in the cavity of the staple holder.

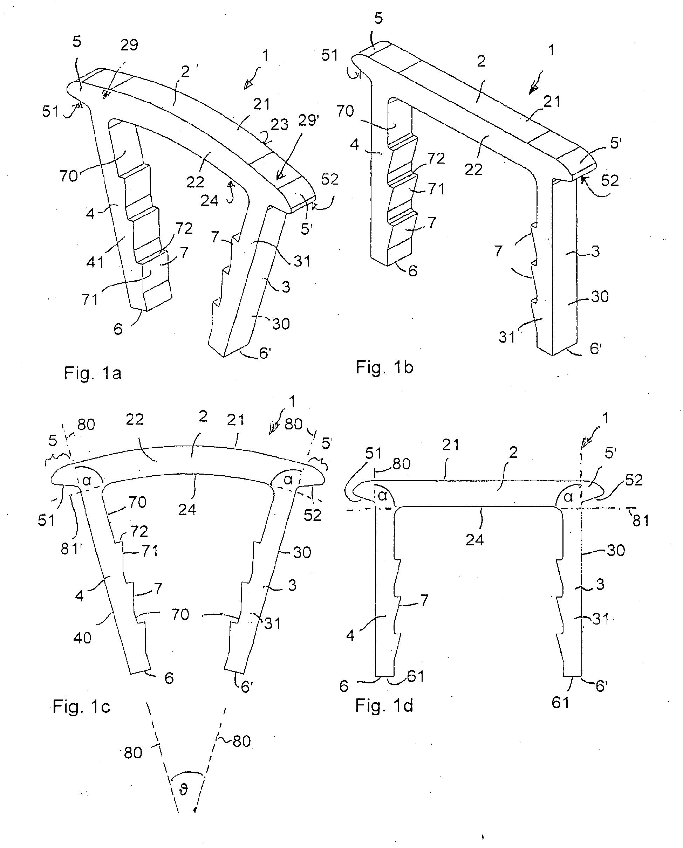

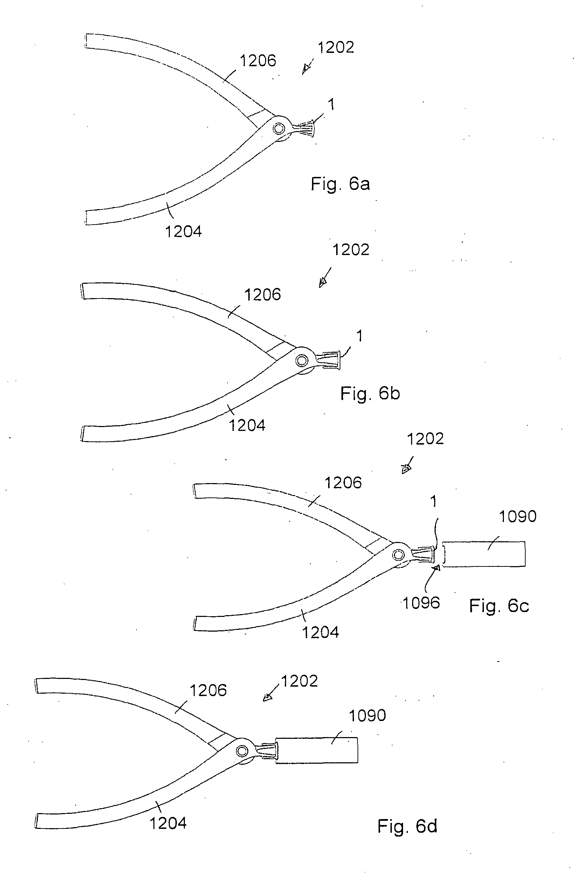

Further embodiments and advantages of the invention will be better understood in view of the following description taken in conjunction with the accompanying drawings. The embodiments which form part of the invention are illustrated in the figures 1a-1d, 3a-3b, 4-7b, 8a-8h, 9a-9d, 18a-18d, 19a-19c, 21, 22a-22c, 23a-23b, 24a-24d, 25a-25d, and 26. The examples shown in the other figures do not form part of the invention but represent background art that is useful for understanding the invention.
- Fig. 1a: shows a perspective view of a surgical staple in a relaxed state according to a first embodiment of a staple with extensions;
- Fig. 1b: shows a perspective view of the surgical staple of Fig. 1a in an expanded state according to the first embodiment;
- Fig. 1c: shows a front view of the surgical staple of Fig. 1a in a relaxed state according to the first embodiment;
- Fig.1d: shows a front view of the surgical staple of Fig. 1a in an expanded state according to the first embodiment;
- Fig. 2a: shows a perspective view of an expansion device according to a first example of an instrument;
- Fig. 2b: shows a perspective view of an expansion device according to an alternative example;
- Fig. 3a: shows a perspective view of a staple holder according to the first embodiment;
- Fig. 3b: shows an enlarged view with regard to a cavity of the staple holder of Fig. 3a;
- Fig. 4: shows an enlarged perspective view of a surgical staple according to the first embodiment received in a relaxed state on the expansion device of Fig. 2a and received in the staple holder of Fig. 3a, which is connected to the expansion device, in an expanded state;
- Fig. 5a: shows a plier according to a second example of an instrument in a state receiving a staple of the first embodiment;
- Fig. 5b: shows a plier according to the second example of an instrument in a state expanding the staple of the first embodiment;
- Fig. 5c: shows a plier according to the second example of an instrument in a state attaching the staple of the first embodiment to the staple holder similar to that of the first embodiment;
- Fig. 6a: shows a plan view of the plier as shown in Fig. 5a, the staple being in a relaxed state;
- Fig. 6b: shows a plan view of the plier as shown in Fig. 5a but the staple being expanded;
- Fig. 6c: shows a plan view of the plier and the staple holder shown in Fig. 5b;
- Fig. 6d: shows a plan view of the plier and the staple holder shown in Fig. 5c;
- Fig. 7a: shows an enlarged perspective view of Fig. 5a;
- Fig. 7b: shows an enlarged perspective view of Fig. 5c;
- Fig. 8a: shows a first step of providing the staple according to the first embodiment in a method of anchoring the staple in a bone (Hallux Valgus);
- Fig. 8b: shows a second step of the method with engaging the engagement portions of the staple via a third embodiment of an instrument according to the invention;
- Fig. 8c: shows a third step of the method with expanding the staple using the instrument of the third embodiment;
- Fig. 8d: shows a fourth step of the method with aligning legs of the staple held expanded by the instrument of the embodiment with holes previously drilled in the bone;
- Fig. 8e: shows a fifth step of the method with inserting the legs into the pre-drilled holes of the bone;
- Fig. 8f: shows an enlarged partially transparent perspective view of the situation depicted in Fig. 8e;
- Fig. 8g: shows an enlarged partially transparent side view of the situation depicted in Fig. 8e;
- Fig. 8h: shows an enlarged partially transparent side view of a situation after a step of releasing the bridge and removing the instrument from the staple;
- Fig. 9a: shows a perspective view of a surgical staple in a relaxed state according to a second embodiment of a staple having asymmetrically extending legs;
- Fig. 9b: shows a perspective view of the surgical staple of Fig. 9a in an expanded state according to the second embodiment;
- Fig. 9c: shows a front view of the surgical staple of Fig. 9a in a relaxed state according to the second embodiment;
- Fig.9d: shows a front view of the surgical staple of Fig. 9a in an expanded state according to the second embodiment;
- Fig. 10a: shows a perspective view of a surgical staple in a relaxed state according to a third example of a staple having a bridge with a T-shaped profile and engagement portions connected on both sides of the bridge;
- Fig. 10b: shows a perspective view of the surgical staple of Fig. 9a in an expanded state;
- Fig. 10c: shows a front view and a side view of the surgical staple of Fig. 9a in a relaxed state;
- Fig.10d: shows a front and a side view of the surgical staple of Fig. 9a in an expanded state;
- Fig. 11a: shows a perspective view of a surgical staple in a relaxed state according to a fourth example of a staple having a bridge with a T-shaped profile and engagement portions separated on both sides of the bridge;
- Fig. 11b: shows a perspective view of the surgical staple of Fig. 11a in an expanded state;
- Fig. 11c: shows a front view of the surgical staple of Fig. 11a in a relaxed state;
- Fig. 11d: shows a front view of the surgical staple of Fig. 11a in an expanded state;
- Fig. 12a: shows the surgical staple of Fig. 11a in a step of providing the staple in a method of anchoring the staple
- Fig. 12b: shows a second step of the method with engaging the staple using a fourth example of an instrument;
- Fig. 12c: shows a third step of the method with expanding the staple using the instrument according to Fig. 12b;
- Fig. 13a: shows a perspective view of a surgical staple in a relaxed state according to a fifth example of a staple having a bridge with a T-shaped profile and engagement portions separated on one side of the bridge while connected on the other side;
- Fig. 13b: shows a perspective view of the surgical staple of Fig. 13a in an expanded state;
- Fig. 13c: shows a front view of the surgical staple of Fig. 13a in a relaxed state;
- Fig.13d: shows a front view of the surgical staple of Fig. 13a in an expanded state;
- Fig. 14a: shows a perspective view of a surgical staple in a relaxed state according to a sixth example of a staple having a bridge with an L-shaped profile;
- Fig. 14b: shows a perspective view of the surgical staple of Fig. 14a in an expanded state;
- Fig. 14c: shows a front and side view of the surgical staple of Fig. 14a in a relaxed state
- Fig.14d: shows a front and side view of the surgical staple of Fig. 14a in an expanded state
- Fig. 15a: shows a perspective view of a surgical staple in a relaxed state according to a seventh example of a staple having a bridge with an L-shaped profile and engagement portions separated from each other on one side of the bridge;
- Fig. 15b: shows a perspective view of the surgical staple of Fig. 15a in an expanded state;
- Fig. 15c: shows a front and side view of the surgical staple of Fig. 15a in a relaxed state;
- Fig.15d: shows a front and side view of the surgical staple of Fig. 15a in an expanded state;
- Fig. 16a: shows a perspective view of a surgical staple in an expanded state according to an eighth example with inclined engagement portion provided on opposite side walls of the bridge;
- Fig. 16b: shows a side view of the surgical staple of Fig. 16a in an expanded state;
- Fig. 17a: shows a perspective view of a surgical staple in an expanded state according to a ninth example with engagement portions formed as recesses at the side walls;
- Fig. 17b: shows a side view of the surgical staple of Fig. 17a in an expanded state;
- Fig. 18a: shows a perspective view of a surgical staple in a relaxed state according to a tenth embodiment of a staple having a bridge with extensions and a discontinuous arcshape;
- Fig. 18b: shows a perspective view of the surgical staple of Fig. 18a in an expanded state according to the tenth embodiment;
- Fig. 18c: shows a front and side view of the surgical staple of Fig. 18a in a relaxed state according to the tenth embodiment;
- Fig.18d: shows a front and side view of the surgical staple of Fig. 18a in an expanded state according to the tenth embodiment;
- Fig. 19a: shows a staple according to the first embodiment in a first step of providing the staple in a method for anchoring a surgical staple;
- Fig. 19b: shows a second step of the method with using a fifth embodiment of an instrument according to the invention to receive the staple in a relaxed state;
- Fig. 19c: shows a third step of the method wherein the staple is expanded using a pressing member.
- Fig. 20a: shows a perspective view of a surgical staple in a relaxed state according to an eleventh example the staple having a bridge with a T-shaped profile and a convexshaped engagement portion;
- Fig. 20b: shows a perspective view of the surgical staple of Fig. 20a in an expanded state;
- Fig. 20c: shows a front and side view of the surgical staple of Fig. 20a in a relaxed state;
- Fig.20d: shows a front and side view of the surgical staple of Fig. 20a in an expanded state;
- Fig. 21: shows a perspective view of a plier according to a sixth embodiment of an instrument in a state of holding an expanded staple of the first embodiment, the plier having an inner screw member to maintain the expanded state;
- Fig. 22a: shows an enlarged view of tip portions of the plier of Fig. 21 in a first step of a method for receiving, expanding, holding and placing a surgical staple according to the first embodiment in a bone;
- Fig. 22b: shows the plier of Fig. 22a in a second step;
- Fig. 22c: shows the plier of Fig. 22a in a third step;
- Fig. 23a: shows a perspective view of a plier according to a seventh embodiment of an instrument in an opened state, the plier having a toggle lever;
- Fig. 23b: shows an enlarged view of the tip portion of the plier of Fig. 23a;
- Fig. 24a: shows in a perspective view the tip portion of the plier of Fig. 23b in a first step of a method for receiving, expanding, holding and placing a surgical staple according to the first embodiment in a bone, in a state of receiving the staple in a cavity;
- Fig. 24b: shows in a partial transparent view of the tip portion of the plier of Fig. 24a details of the toggle lever;
- Fig. 24c: shows a side view of the tip portion of the plier of Fig. 24a;
- Fig. 24d: shows a plan view of the tip portion including a bottom handle, a toggle joint and the cavity of the plier of Fig. 24a;
- Fig. 25a: shows in a perspective view the tip portion of the plier of Fig. 23b in a second step of a method for receiving, expanding, holding and placing a surgical staple according to the first embodiment in a bone, in a state of expanding and holding the staple in the cavity;
- Fig. 25b: shows in a partial transparent view of the tip portion of the plier of Fig. 25a details of the toggle lever;
- Fig. 25c: shows a side view of the tip portion of the plier of Fig. 25a;
- Fig. 25d: shows a plan view of the tip portion including a bottom handle, a toggle joint and the cavity of the plier of Fig. 25a;
- Fig. 26: shows a schematical drawing indicating the distribution of compression forces at height levels along the leg in the expanded state of the surgical staple in Figs. 8g or when just released as shown in Fig. 8h .

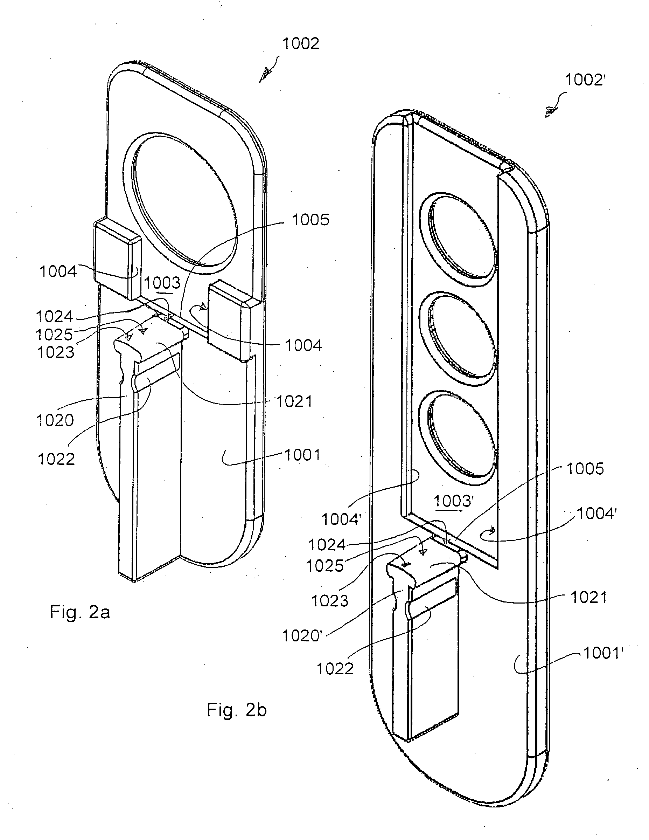

A first embodiment of a surgical staple 1, which can be used for example with embodiments of an expansion device 1002 and a staple holder 1090 shown in Figs. 2a through 4, respectively, is explained with reference to Figs. 1a - 1d. Figs. 1a and 1c reveals details of the surgical staple 1 in a relaxed state in perspective and front views, respectively. A relaxed state is defined herein as a state in which no external forces act on the staple. In case that the staple is at least partially made from shape memory materials, this state further refers an austenite phase state, wherein the shape attained in this state is related to the shape memorized upon fabrication.

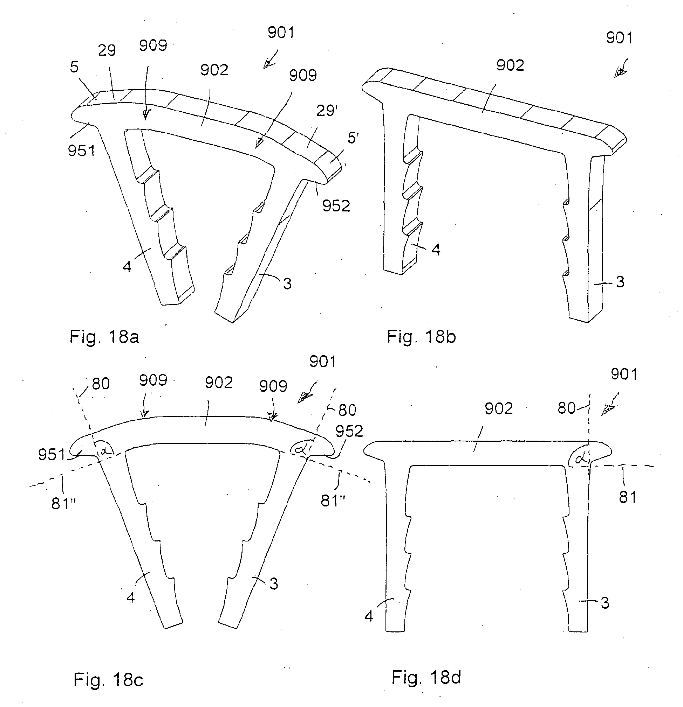

The surgical staple comprises a bridge 2, a first leg 3 and a second leg 4 both connected to the bridge 2 at respective end sections 29, 29'. The bridge 2 includes an elongate body extending between and including end sections 29, 29'. The bridge 2 in this embodiment has a rectangular cross section thereby forming a top surface 21, front and back side surfaces 22, 23 and bottom surface 24. In the relaxed state shown in Figs. 1a and 1c, the bridge 2 is arc-shaped or curved. The curvature, or radius of curvature is constant in the embodiments shown but may also vary along the length of the bridge, or may be present only in portions thereof (an example thereof will be explained with reference to Figs. 18a-18d below).

The legs 3 and 4 extend from the end sections 29, 29' including an angle α of about 90° with respect to the bridge at the location of the connection, respectively, as can be seen in Fig. 1c. The bottom surface 24 of the arc-shaped bridge 2 forms a curved plane 81' in the relaxed state and the angle α may for example be measured at an intersection of a line or plane 80 extending along lateral surfaces 30, 40 of legs 3, 4, respectively, and curved plane 81' as illustrated in Fig. 1c. As will be described in more detail below, angles α do not (substantially) vary when the staple changes from the relaxed to the expanded state.

In this first embodiment, as depicted in Figs. 1a - 1d, the end sections 29, 29' of the bridge 2 include extension portions 5, 5' which extend beyond the location of connection between the bridge 2 and the legs 3, 4, respectively. The extension portions 5, 5' are both tapered towards respective rounded tips, thereby forming slightly inclined surfaces which may be engaged by corresponding engagement structures of an external tool in order to expand, or maintain an expansion of, the surgical staple 1 as will be described below. More specifically, the extensions 5, 5' each form engagement portions 51, 52 according to the invention, which may be received for example in a form-fit manner by an external instrument or tool.

The legs 3 and 4 of the surgical staple 1 have outer lateral side surfaces 30 and 40, inner side surfaces 70 and front/back side surfaces 31 and 41, respectively, and also include a rectangular cross section. At the inner side surfaces a number of barbs 7 is formed which comprise a slightly inclined wall 71 and a perpendicularly protruding wall 72 which forms a sharp edge. The barbs 7 are directed upwardly and serve to improve anchoring in the bone. An upper portion of the legs 3, 4 adjacent the bottom surface 24 of the bridge 2 may be left free of barbs 7. In this specific embodiment, three barbs 7 are formed at the inner surfaces 70 of the legs 3 and 4, but more or less or even no barbs may be formed in other embodiments. Moreover, each of the legs 3, 4 has a tip 6, 6', respectively. In this embodiment, tips 6, 6' are flat surfaces which have the overall square-like cross section of the legs. However, it is also possible that the tips 6, 6' are tapered and/or sharp and/or rounded and/or conical shaped as known in the art in order to improve the insertion process of the legs into the bones. This pertains also to the other embodiments described below.

The legs 3 and 4 extend substantially perpendicularly from the bridge 2 as noted above. As the bridge 2 is arc-shaped in the relaxed state, the legs 3 and 4 consequently extend towards each other. In this specific embodiment, lines or planes 80, 80' extending along the outer lateral side walls 30, 40 of the legs, 3, 4, respectively, intersect each other at about 30° in the relaxed state. However, other intersection angles ϑ between 25° and 35° or between 20° and 40° or even beyond these values are encompassed as well.

Figs. 1b and 1d show an expanded state of the surgical staple 1 of the first embodiment. In this state the legs 3 and 4 are laterally moved away from each other such as to be arranged in parallel with respect to each other. The expansion from the relaxed state is accomplished by applying mechanical energy to the bridge 2 via engaging portions 51, 52, wherein the bridge 2 may be bent like a spring, when conventional materials such as stainless steel are considered for the staple. This applies also when a shape memory material such as Nitinol is considered for the surgical staple. However, the range of deformation can be extended due to the superelasticity of this material.

Alternatively, when a shape memory material such as for example Nitinol is considered for the surgical staple, the expansion may also relate to an elastic deformation of the staple in the martensitic phase by cooling the staple before expansion. The staple then exerts its full compression onto the bones after insertion when the staple is again at a higher temperature (body temperature).

As can be seen particularly in Fig. 1d, the angles α are maintained during the expansion of the surgical staple 1 at 90° with respect to both legs 3 and 4. As a consequence of the legs 3, 4 being made parallel, the bridge 2 attains a straight shape, and its bottom surface 24 becomes substantially flat, i.e., almost not curved. The bottom surface 24 thus defines a straight plane 81 perpendicular to a plane defined by the bridge 2 and the legs 3 and 4.

In the expanded state shown in Figs. 1b and 1d, the inclined surfaces of the engagement portions 51 and 52 remain being arranged above the plane 81 defined by the bottom surface 24 of the bridge 2 and directions perpendicular to legs 3, 4, or axis 80, respectively, which may be associated with the bone surface, when the staple is implanted to the bone(s). When the surgical staple 1 is attached to bones with the legs 3 and 4 being for example inserted into holes drilled in advance into respective bones or bone fragments, the insertion process may be made until the bottom surface 24 of the bridge abuts on the plain bone surface, since the engagement portions 51 and 52 may advantageously be engaged or disengaged above that surface.

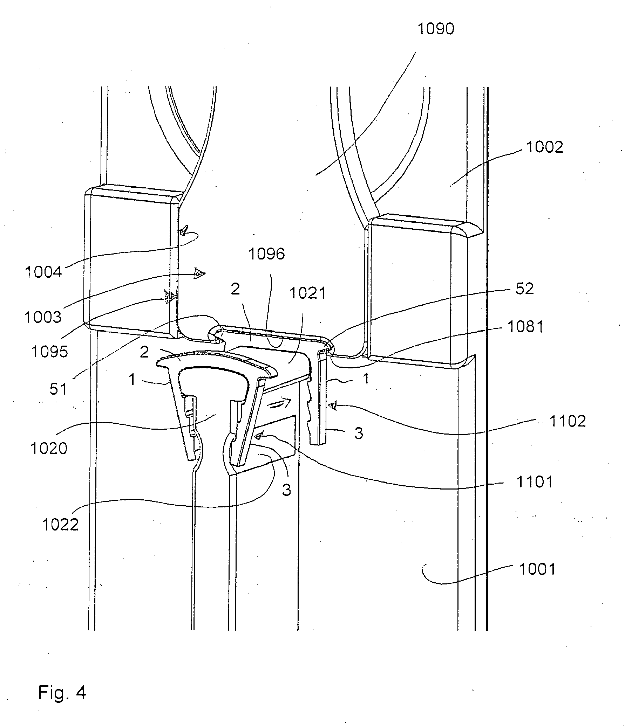

An example of a device 1002 for expanding the surgical staple 1 is displayed in Fig. 2a. The expansion device 1002 is non-limiting and other devices may be used to effect expansion of the surgical staple 1. The expansion device 1002 shown has a substantially plate-shaped body 1001 from which protrudes a boss 1020, which is removably connected to the body 1001 and is arranged to receive the surgical staple 1 in a relaxed state 1101 (see Fig. 4) at a first portion 1023 adjacent the free end of the boss 1020. An upper surface 1021 of the boss 1020 is designed to contact the bridge 2, and more specifically: the bottom surface 24 thereof. Consequently, in the first portion 1020, the upper surface 1021 has a curvature corresponding to the arc-shape of the bottom surface 24 or the curved plane 81' as shown in Fig. 1b. The legs 3 and 4 extending towards each other in the relaxed state are received in recesses 1022 formed in the boss 1020 on lateral faces thereof below the upper surface 1021.

The boss 1020 also has a second portion 1024 adjacent the plate-shaped body 1001 from which the boss 1020 extends. In the second portion 1024, the upper surface is almost flat corresponding the flatness of plane 81 of the bottom surface 24 in an expanded state 1102 of the surgical staple 1 as shown in the enlarged view of Fig. 4. Also, in the second portion 1024 of the boss 1020, which portion is thus configured to receive the surgical staple 1 in the expanded state, the depth of recesses 1022 for receiving the legs 3, 4 is decreased.

Between the first portion 1023 of the boss 1020 for receiving the surgical staple 1 in the relaxed state 1101 and the second portion 1024 of the boss 1020 for receiving the surgical staple 1 in the expanded state 1102, there is provided a transfer portion 1025, in which the profile of the upper surface 1021 smoothly changes from curved to flat, and the depth of the recesses 1022 smoothly varies from deep to shallow. Hence, transfer portion 1025 allows continuous expansion of the staple 1 by (a) attaching the staple to the boss 1020 at the first portion 1023, (b) displacing the staple 1 along the transfer portion 1025, and (c) disposing the staple 1 at the second portion 1024 adjacent the plate-shaped body 1001.

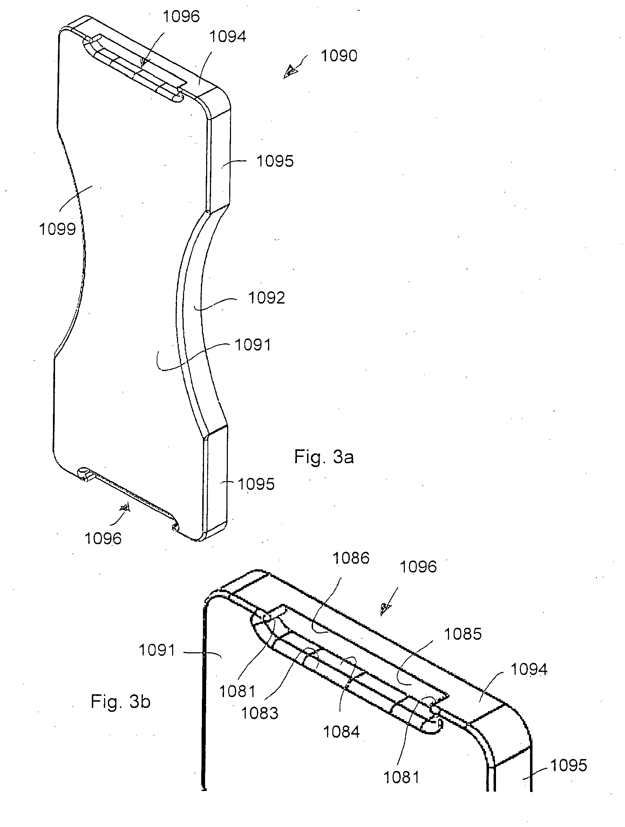

The plate-shaped body 1001 is provided with guide walls 1004 and an abutment wall 1005 which form an accommodation space 1003 for accommodating a staple holder 1090, which is shown in Figs. 3a and 3b.

The staple holder 1090 has a flat, substantially rectangular plate-shaped body 1099 with opposite flat faces 1091 and narrow side faces 1095, 1096 and narrow front faces 1094. Narrow side faces 1095, 1096 involve a recess 1092 configured to allow safe manual handling of staple holder. The narrow front faces 1094 respectively include apertures 1086 allowing access to cavities 1096 formed adjacent to the front faces 1094. The cavities 1096 are configured (i.e., shaped, sized and dimensioned) to receive the bridge 2 of a respective staple 1 as that shown with respect to the first embodiment.

Moreover, the cavities 1096 are formed with engagement structures 1081, respectively, which are provided at corresponding ends of a cavity 1096 associated with the locations of the engagement portions 51, 52 of the bridge 2 when received therein. The engagement structures 1081 are in this embodiment small projections (narrowing aperture 1086 with respect to cavity 1096) inserted below the engagement portions 51, 52 of the bridge 2, when the staple is displaced into the second portion of the boss 1020, and hence allow to engage the engagement portions 51 and 52 and thus to maintain the expanded state 1102 of the surgical staple 1 when received in one of the cavities 1096, as it is depicted in Fig. 4. A counterforce is necessarily exerted onto the top surface 21 of the bridge 2 particularly in a centre portion thereof, and may for example be provided by an inner back wall opposite the aperture 1086 of the cavity, which back wall herein serves as a pressing portion 1084.

As shown in Fig. 4, the accommodation space 1003 provided by guide and abutment walls 1004 and 1005 allows accommodating the staple holder 1090 such that a second aperture 1083 formed in one of the flat faces 1091 adjacent the front faces 1094 allows inserting the bridge 2 when travelling into the second portion 1024 of the boss 1020. When the bridge is in the second portion 1024 with the legs 3, 4 abutting on the plate-shaped body 1001 of the expansion device 1002, the bridge 2 coincidently abuts on a side wall 1085 opposite the aperture 1083 of the cavity 1096.

Having once received the bridge 2 in one of the cavities 1096, the boss 1020 can be removed from the body 1001, and the staple holder 1090 may be removed from the expansion device 1001 with the surgical staple 1 held in the expanded state by virtue of engagement structures 1081 and engagement portion 51, 52, respectively, and further by virtue of a pressing force exerted by pressing portion 1084 urging into a direction opposite to the pulling force of the engagement structures.

Next, the surgical staple 1 may be attached to a bone or bone assembly with holes pre-drilled therein (not shown in this embodiment). Since the legs 3, 4 are held by the staple holder 1090 in parallel, manual insertion is facilitated until the bridge 2 abuts on the bone surface. In a next step the staple holder 1090 may release the bridge by a lateral movement, wherein the bridge 2 already adhering to the bone surface leaves the cavity 1096 through the lateral second aperture 1083. As a consequence, the bridge 2 is released, which thus tries to return into its original curved shape in view of the mechanical energy stored therein, whereby a compression force is exerted on the legs 3, 4, which adversely compress the bones or bone fragments involved via the pre-drilled holes in which the legs are inserted.

Advantageously, no further hammering-in of the staple is necessary according to this embodiment and the embodiments described below. As a consequence, damage of the bone structure inside the pre-drilled holes in the region of the teeth may be avoided.

A further advantage of this and also the other embodiments described herein arises in that since the mechanical energy is stored in the initially arc-shaped or curved bridge 2 which is then made to have a straight shape, a distribution of the compression force (of the legs 3, 4 towards each other) along the legs 3, 4 is distributed in a broadened manner and thus improved, and may have a maximum in a region including the first and second barbs 7 when counted from the side of the bridge 2. Moreover, the compression forces are distributed farther away from the bridge 2, i.e., act upon the inner walls 1352 of the holes 1351, which are formed in the bone 1350 when the staple 1 is inserted therein, at a deeper position. Hence, the reliability of the anchoring is also considerably improved.

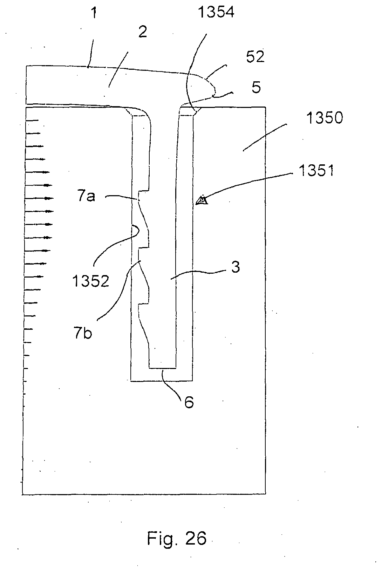

An impression of the distribution of compression forces exerted by the leg 3 of the surgical staple 1 is provided in Fig. 26 as an example. The surgical staple is placed in a pre-drilled hole 1351 formed in a bone 1350. The arrows indicate the amount of force exerted by a respective portion of the leg 3 at a given height level, the force being oriented in a direction parallel to the bridge 2 and perpendicular to the legs 3, 4, when the surgical staple 1 is in the expanded state, or is still expanded and just released to exert the forces onto the inner wall 1352 of the hole 1350.

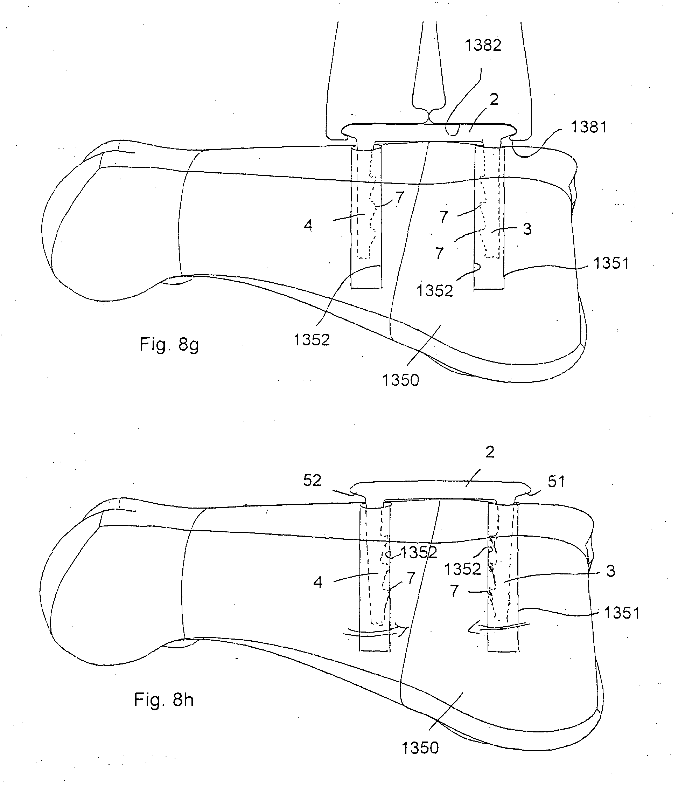

The situation is depicted for example in Figs. 8g or 8h as explained below. As can be seen from Fig. 26, a maximum compression force is exerted at about a height level of the first barb 7a, or between the first barb 7a and the second barb 7b, while a smooth distribution of forces up the tip 6, 6' of the leg 3 is achieved. According to the embodiments disclosed herein, a smooth maximum is achieved in a middle third or even in a bottom third along the length of a leg. In the known art, a more sharp peak is generally measured at a height level of a leg closer to an upper surface of the bone, or closer to the bridge, i.e. in an upper third.

Still further, by storing the mechanical energy within and along the bridge, stress and strain may particularly be reduced at one of the most critical point of the staple, the inner corner at the junction, or location of connection, between the legs 3, 4 and the bridge 2. This may further improve the reliability of the staple.

The above described advantages also apply to the embodiments described in the following. One particular advantage of the first (and second) embodiment of the surgical staple is that due to the engagement portions formed as projections at the end section 29, 29' beyond the junction, namely by means of extensions 5, 5', the moment of torque for bending the bridge is considerably increased as compared to a case where for example the bottom surface 24 of the bridge is engaged adjacent the connection with the legs 3, 4. This in turn relaxes the requirements for the external instrument to maintain the expanded state of the staple 1, 101.

In the above description the staple holder 1090 along with the expansion device 1002 forms a first embodiment of an instrument.

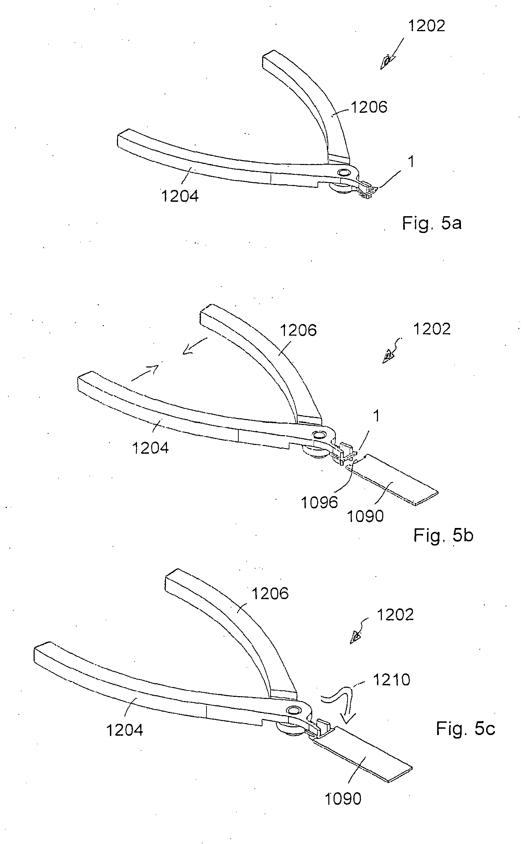

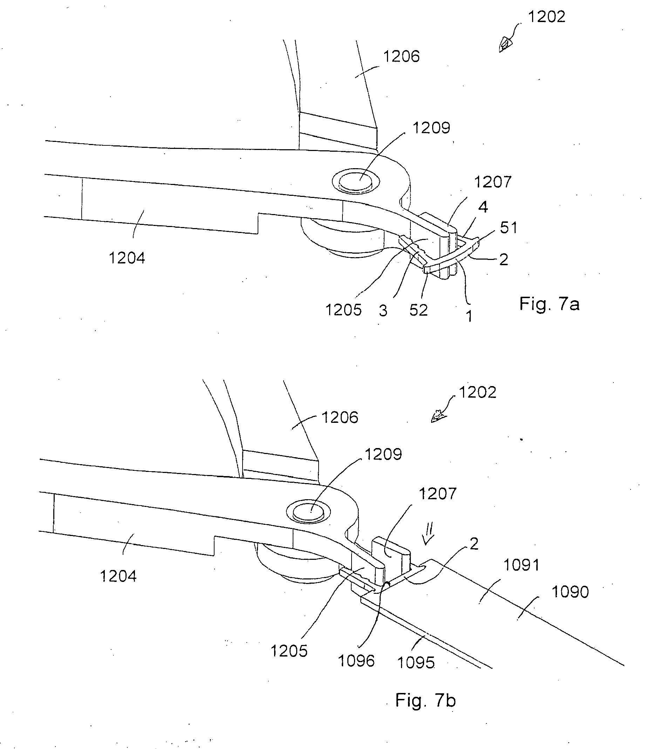

As shown in Figs. 5a - 7b, a second example of an instrument is shown which can be used in conjunction with staples 1, 101 and 901 of the first, second and tenth embodiment (described below) of a surgical staple. In this embodiment, the expansion device is represented by a plier 1202, while the staple holder 1090 of the first embodiment (instrument) can be used herein as well to receive and hold the staple 1, 101. In the drawings of Figs. 5a - 7b, walls 1092 and 1085 are omitted for simplicity. The plier 1202 includes two handles 1204, 1206 rotatably coupled via shaft 1209. Each handle 1204, 1206 has a tip portion 1205, 1207, respectively. As shown in more detail in Fig. 7a, in a first position of the plier 1202 where the handles 1202, 1204 are opened, the tip portions 1205, 1207 are correspondingly closed such that legs 3, 4 of the staple may be received on an outer side thereof. Moving the handles then into a closed configuration corresponding to a second position of the plier 1202, the tip portions 1205, 12β7 move away from each other such as to splay the legs 3, 4 of the surgical staple 1 as shown in Fig. 7b.

In this expanded state, the plier may be arranged with respect to the staple holder 1090 such that the bridge 2 is inserted into the cavity 1096 through the lateral aperture 1083 with the legs protruding from the cavity 1096 through a frontal aperture 1086 of the staple holder 1090. The plier 1202 manually held until this step under tension, may then be released. As a consequence, the engaging portions 51, 52 of the surgical staple are forcibly engaged by the engaging structures 1081 of the staple holder 1090 and a pressing force is exerted by the pressing portion 1084 of the back wall of the cavity 1096. The plier may then be removed, and the surgical staple placed in a bone as explained above using the staple holder 1090.

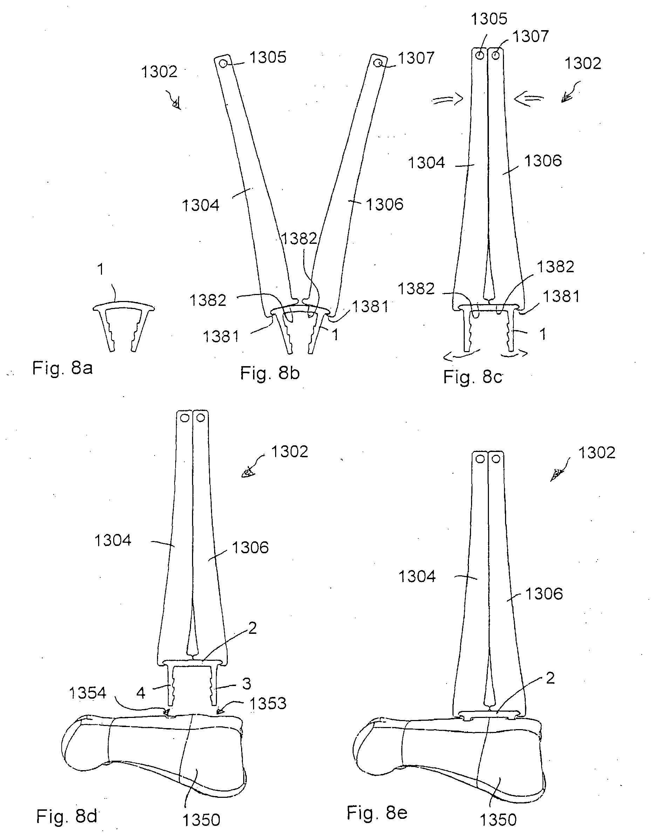

A third embodiment of an instrument according to the invention and a method of using the same are described with reference to Figs. 8a - 8h. The tasks of expanding and holding the staple are performed by one single device different from the instrument according to the first and second embodiments.

Fig. 8a shows a step of providing the surgical staple 1. Staples 101 or 901 provided with extensions 5, 5' as engagement portions 51, 52, 151, 152, 951, 952 are compatible with this instrument as well.

Fig. 8b shows a second step of engaging the surgical staple 1 by means of plier instrument 1302, i.e., the bridge 2 including the engaging portions 51, 52 of staple 1 is received between the engagement structures 1381 provided at the tips of handles or arms 1304, 1306. Plier instrument 1302 has two such handles or arms 1304, 1306, which have interfaces 1305, 1307, respectively, for connecting further plier mechanics (not shown) allowing to suitably actuate the arms 1304, 1306 by for example manual input. Such mechanics are well-known in the field of pliers and serve to provide a cooperating movement of the arms.

Fig. 8c shows a third step of the method, wherein the surgical staple 1 is expanded by closing the arms 1304, 1306 of the plier instrument 1302 thereby pulling the engaging portions 51, 52 upwards and pressing a centre portion of the top surface 21 downwards by means of the pressing portion 1382 also provided at the tip portion of the arms 1304, 1306, respectively. In this state, by the joint movement of the arms 1304, 1306, a cavity is dynamically formed by means of engagement structures 1381 and pressing portions 1382 which holds the bridge 2 in a bent and staple 1 in an expanded state.

Fig. 8d illustrates a fourth step of applying the expanded surgical staple 1 to the situation of a hallux valgus. In this specific, non-limiting example, an AKIN procedure involving a surgical correction of a misalignment of the os metatarsalis is performed via osteotomy. The surgical staple 1 is applied to holes 1353, 1354 previously drilled into the fragments of the bone 1350 (in this example: os metatarsalis). The legs 3, 4 of the staple are aligned with such holes 1353, 1354, respectively, using the plier instrument 1302.

Fig. 8e shows a fifth step of the method wherein the legs 3, 4 are fully inserted into the holes 1353, 1354 using the plier instrument 1302. The bottom surface 24 of the bridge 2 has engaged the bone surface, while the engagement structures 1381 are still in forcible engagement with the bridge 2.

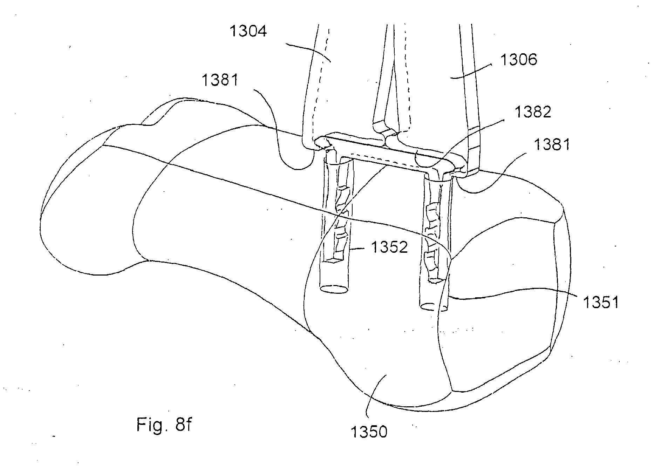

Fig. 8f shows a perspective view of the situation depicted in Fig. 8e, wherein the legs 3, 4 are residing in the holes 1353, 1354 parallel to each other in the expanded state held by the plier instrument 1302.

Fig. 8g shows a side view of the situation depicted in Fig. 8f. The legs 3, 4 have not yet engaged the side walls of the holes 1353, 1354 and the staple is thus not yet anchored in the bone 1350.

Fig. 8h shows a sixth step of the method of using the plier instrument 1302. The bridge 2 has now been released by the plier instrument 1302, which is now removed. No direct forces act on the bridge 2 any more. Hence, the bridge 2 starts to release the mechanical energy stored thereby tending to return back into the arc-shape. Occasionally, the legs 3, 4 moving towards each other abut on mutually opposite side wall surfaces of the holes 1353, 1354 thereby compressing the two bone fragments of bone 1350. As the barbs 7 bite or dig into the bony side wall surfaces of holes 1353, 1354, anchoring is also accomplished thereby.

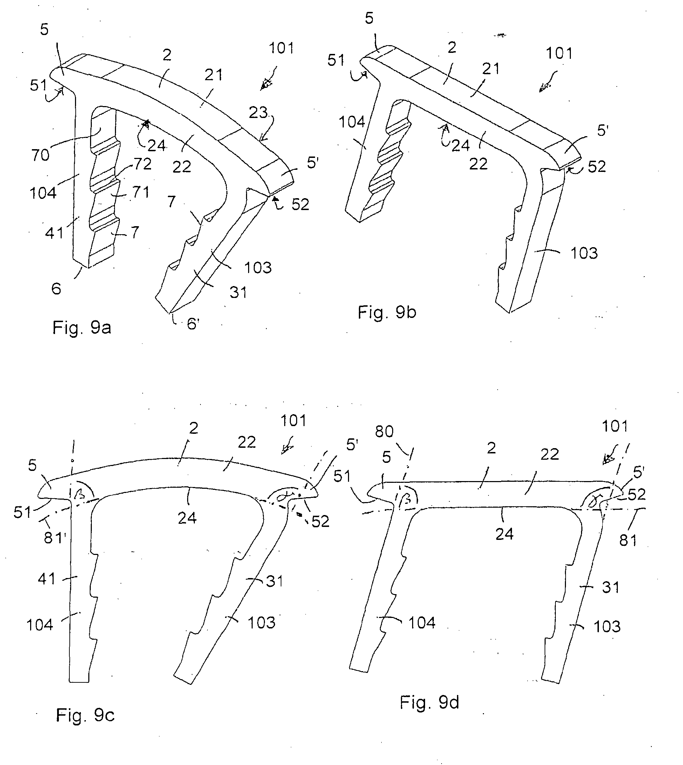

In the following, a modification of the first embodiment of a surgical staple will be described with regard to a surgical staple 101 according to second embodiment with reference to Figs. 9a-9d. Same or similar features are denoted with the same numerals and repeated description thereof will be omitted.

The second embodiment of a staple differs from the first embodiment in that the legs 103, 104 extend from the bridge 2 at angles β and γ, respectively, which are different from 90°. More specifically, both legs 103, 104 do not extend from end sections 29, 29' of the bridge 2 perpendicularly, but at an oblique angle. Both angles differ from 90° by about 15°. The inclination is made to be oriented towards the same direction (in Figs. 9a - 9d, towards the left side direction). As a consequence, as can be seen in Figs. 9b and 9d, in the expanded state, both legs are inclined with respect to the bridge 2 thereby extending in parallel to each other.

The embodiment becomes particularly advantageous in clinical situations where an inclined insertion is necessary.

Further embodiments described in the following differ from the first and second embodiments in that the engagement portions - while still being provided in or adjacent the end sections 29, 29' - are arranged on the side wall surfaces of respective bridges. In what follows, same or similar features as in the first embodiment are denoted with the same numerals and description thereof will be omitted.

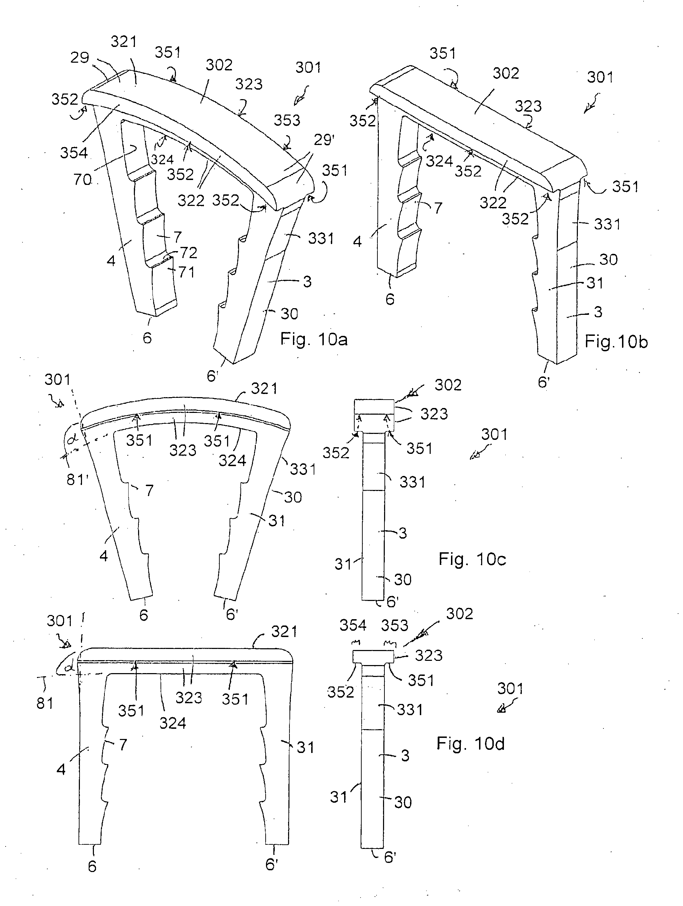

For example, a third example of a surgical staple 301 is depicted in Figs. 10a - 10d. In this example, a bridge 302 is provided which has a top surface 321, a front side wall surface 322, a back side wall surface 323 and a bottom surface 324 (the terms "front" and "back" simply make reference to the drawings herein, of course the staple may be reversed). As in the first and second embodiments, the bridge 302 of surgical staple 301 is arc-shaped or curved in the relaxed state as shown in Figs. 10a and 10c, and is straight or plane-shaped in the expanded state as shown in the Figs. 10b and 10d. The legs 3, 4 are similar to the first embodiment, wherein upper ends adjacent the bridge 302 are slightly reinforced by inclined outer surfaces 331.

At both side wall surfaces 322, 323 there are provided protrusions flush with the top surface 321, which extends along the back and front wall surface from the first end section 29 up to the second end section 29'. The protrusions represent engagement portions 351, 352 which each form an overhang at the back and front wall side surfaces 322, 323 with a surface at its bottom, which is oriented towards the legs 3, 4 and which serves to receive a pulling force from engagement structures of an external instrument (not shown). The bridge 302 thus has, when seen in cross section, a T-shape along almost its entire length. The engagement portions 351, 352 may be engaged by an engagement structure of an external instrument (not shown) to hold the surgical staple 301 in an expanded state.

The protrusions of the engagement portion 351, 352 extend up to the end sections 29, 29' such that the necessary moments of torque to maintain the expanded state of the surgical staple 301 can be afforded by the external instrument. For example, the staple holder 1090 of Figs. 3a, 3b may be modified to include a cavity having engagement structures complementary in shape to the instant protrusions. As in the first and second embodiments, the engagement portion 351, 352 are located above a plane 81 in the expanded state which allows removal of the external instrument even in the fully installed state in the bone.

It should be noted that while the engagement portions 351, 351 extend from one end section 29 to the other end section 29' along the side wall surfaces 322, 323, engagement by engagement structures of an external instrument may occur only in parts thereof, preferably at or adjacent the end section 29, 29', as indicated above.

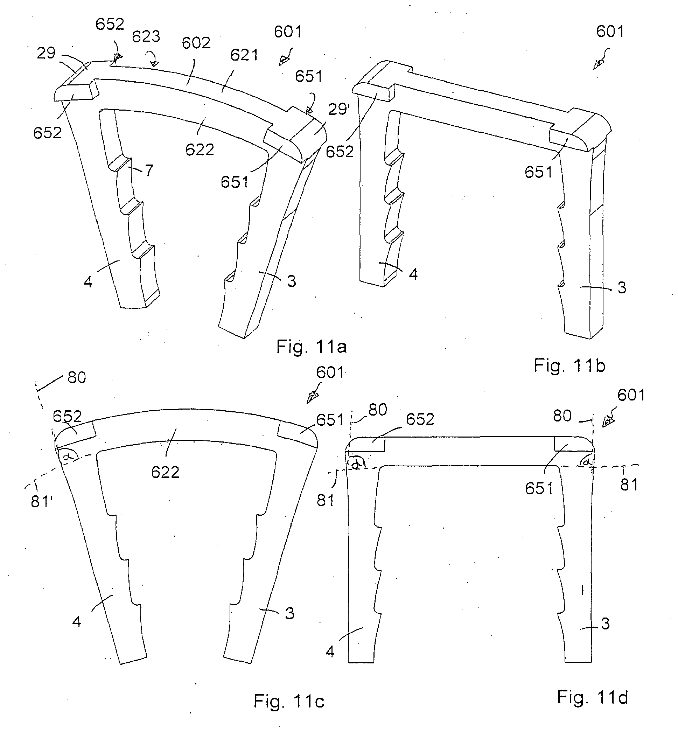

Accordingly, in a fourth example of a surgical staple 601 shown with respect to Figs. 11a - 11d, engagement portions 651, 652 may also be provided to extend along the side wall surfaces 622, 623 only within end sections 29, 29'. Hence, the T-shape of the bridge 602 is also provided only in the end sections 29, 29' of the bridge 602.

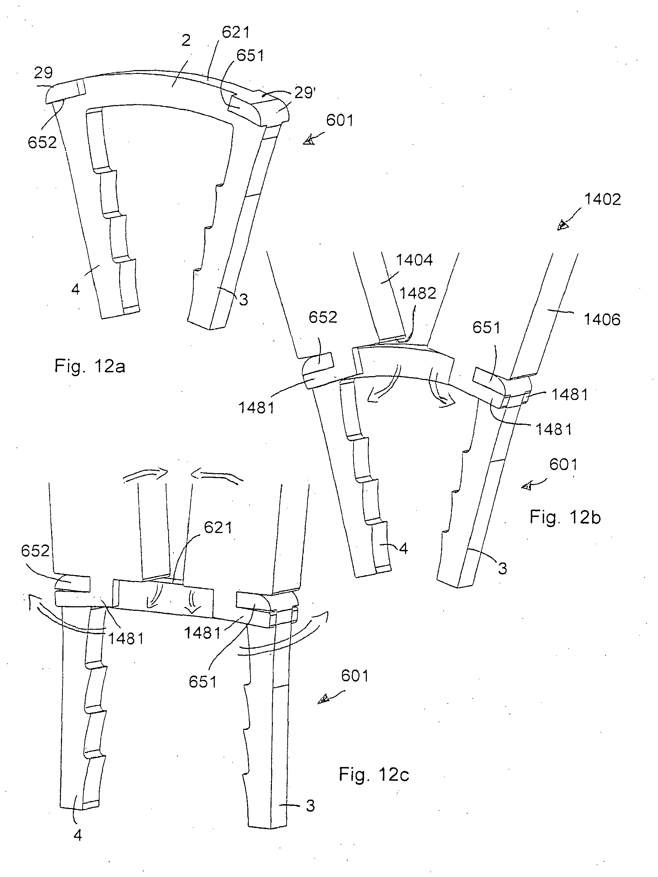

A fourth example of an instrument 1402 according to the invention illustrated with respect to Figs. 12a - 12c may be applied in conjunction with the fourth embodiment of the surgical staple 601. Fig. 12a shows the step of providing the surgical staple 601, while Fig. 12b indicates the instrument 1406 attached to the staple 601. The instrument 1402 may be, for example a plier instrument or spreading pliers or another device. It may have a similar structure of arms or handles 1404, 1406 as that shown with respect to the plier instrument 1302 of Figs. 8a - 8h (third embodiment of an instrument).

The arms or handles 1404, 1406 have engagement structures 1481 extending from tip portions of the arms and engage around the projections of the engagement portions 651, 652 of the bridge 602 from a centre direction of the bridge 602 in a direction outwards away from each other. The handles or arms 1404, 1406 are then closed at an end opposite the tip portions in a third step such that the tip portions itself perform a rotational movement, as it is shown in Fig. 12c, thereby lifting or pulling the engagement portions 651, 652 by virtue of the engagement structures 1481. At the same time, flat pressing portions 1482 also provided at the tip portions of the arms or handles 1404, 1406 press onto the top surface 621 of the bridge, which results in an expansion of the staple. The further steps may be the same as explained with regard to Fig. 8d - 8h.

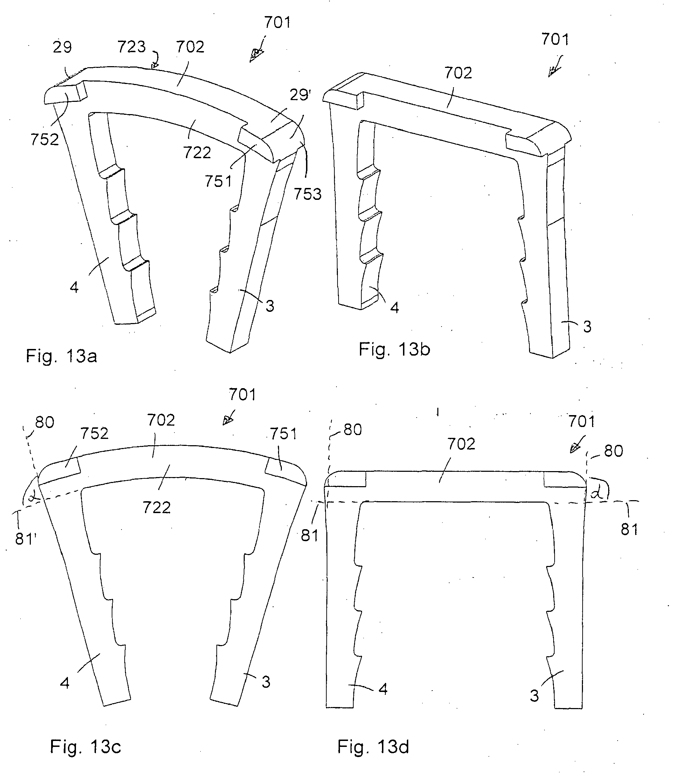

A fifth example of a surgical staple 701 is shown in Figs. 13a - 13d. The surgical staple 701 differs from the staples 301 and 601 in that one (front) side wall surface 722 of the bridge 702 has two projections corresponding to engagement portions 751, 752 provided only at the end sections 29, 29', while the other (back) side wall surface 723 has one projection corresponding to engagement portion 753 fully extending from one end section 29 to the other end section 29'.

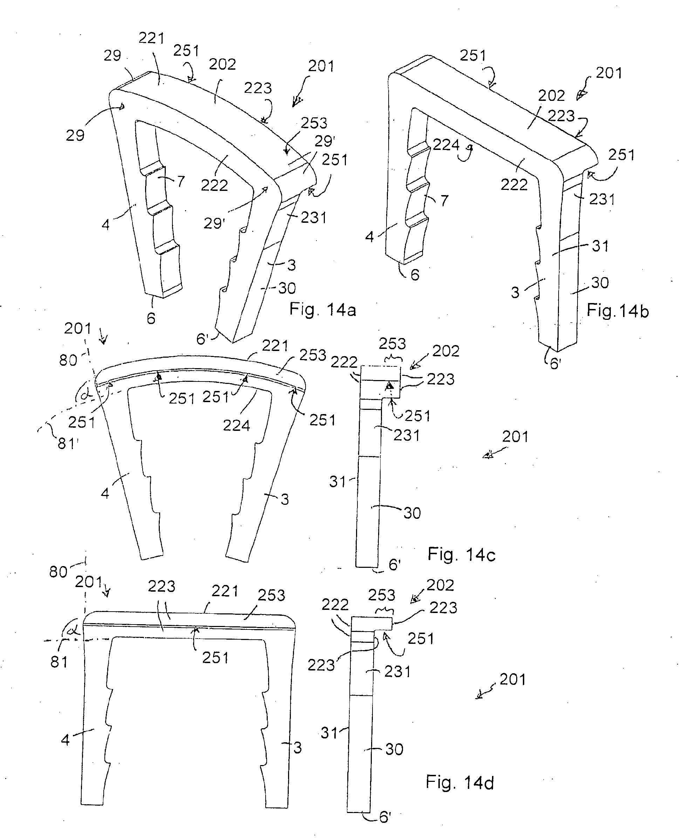

A sixth example of a surgical staple is depicted in Figs. 14a - 14d. In this example, a bridge 202 is provided which has a top surface 221, a front wall surface 222, a back side wall surface 223 and a bottom surface 224 (the terms "front" and "back" simply make reference to the drawings herein, of course the staple may be reversed). As in the previous embodiments, the bridge 202 of surgical staple 201 is arc-shaped or curved in the relaxed state as shown in Figs. 14a and 14c, and is straight or plane-shaped in the expanded state as shown in the Figs. 14b and 14d. The legs 3, 4 are similar to the first embodiment, wherein upper ends adjacent the bridge 202 are slightly reinforced by inclined outer surfaces 331.

Only at the "back" side wall surface 223, there is provided a protrusion flush with the top surface 221, which extends along the back wall surface from the first end section 29 up to the second end section 29'. As in the third to fifth embodiments of a staple, the protrusion 253 forms an engagement portion 251 which may be engaged by an engagement structure of an external instrument (not shown) to hold the surgical staple 201 in an expanded state. The cross section of the bridge 202 according to this embodiment is L-shaped.

The advantages and effects achieved by the sixth embodiment are also similar to that of the third to fifth embodiments. The surfaces of engagement portions 251 are oriented to, or face, the legs 3,4, and are located above a plane 81 defined by the bottom surface 224 of the bridge 202. Legs 3, 4 also include a reinforcement with regard to inclined outer surface 231.

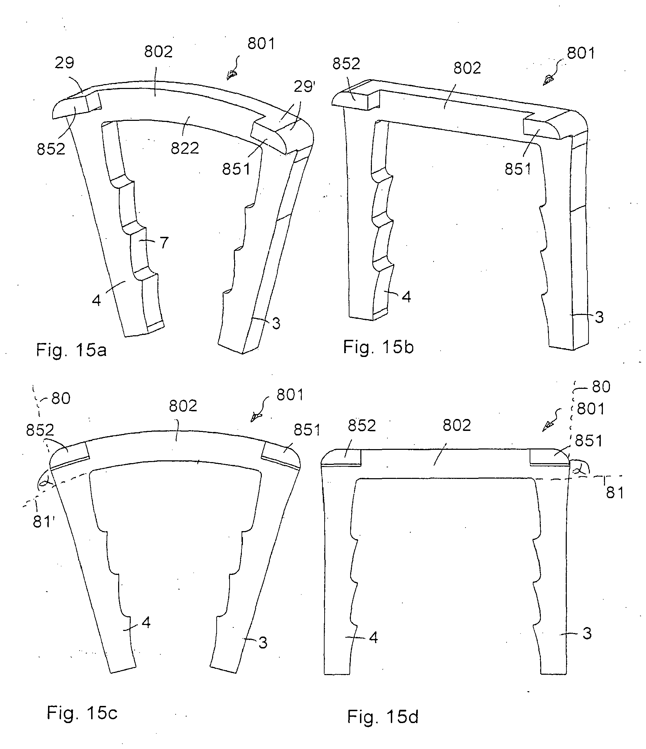

A seventh example of a surgical staple 801 is shown with regard to Figs. 15a - 15d. The surgical staple 801 is a simple modification of that shown in the sixth example, wherein, however, the projection extends only in the end sections 29, 29' to yield single-sided engagement portions 851, 852 extending along the side wall surface 822 only in a limited region.

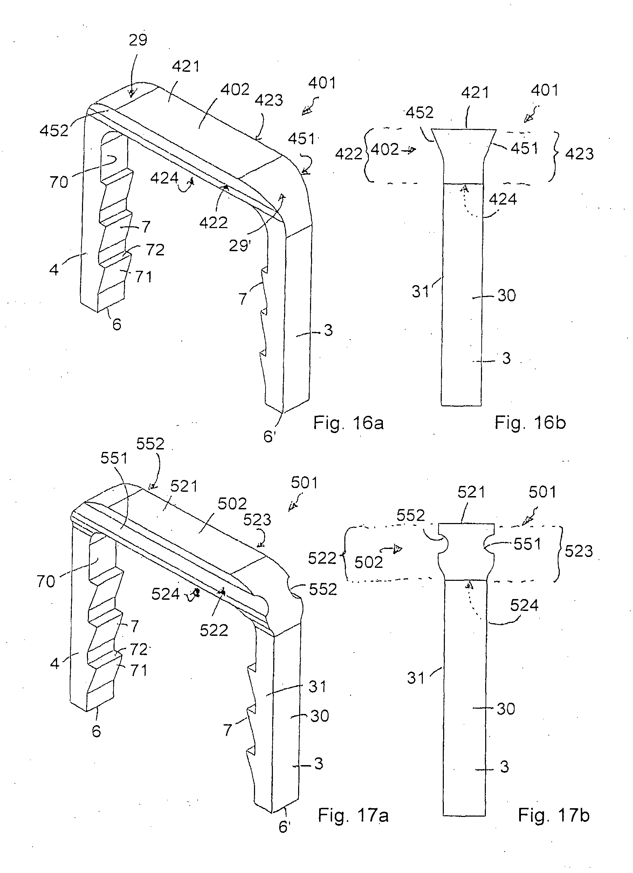

An eighth example will be described with reference to Figs. 16a - 16b, which shows a surgical staple 401 only in an expanded state. A relaxed state (not shown) is analogous to the previous embodiments. This example is similar to the third example in that two continuous protrusions are formed at both of the side wall surfaces 422, 423 of the bridge 402, and are flush with the top surface 421. However, different from the previous embodiment, the protrusions include slightly oblique surfaces, which are only slightly inclined towards the legs 3, 4. These oblique surfaces extend along the bridge 402 from a first end section 29 up the second end section 29' and are located above the plane 81 defined by the bottom surface 424 of the bridge 402. The protrusions and oblique surfaces represent engagement portions 451, 452 according to the invention and may be engaged by corresponding engagement structures of an external instrument (not shown) such as to maintain the expanded state prior and during implantation.

A ninth example will be described with reference to Figs. 17a - 17b, which shows a surgical staple 501 also only in an expanded state. A relaxed state (not shown) is analogous to the previous embodiments. In this example, the side wall surfaces 522 and 523 of the bridge 502 include recesses extending from the first end section 29 up to the second end section 29' at a central height level thereof, i.e., above a plane 81 defined by bottom surface 524 opposite the flat top surface 521. The recesses include a concave cross section and serve as engagement portions 551, 552 to be engaged by engagement structures of an external instrument (not shown) for maintaining the surgical staple 501 in the expanded state shown in Figs. 17a and 17b. Since the recesses reduce the thickness of the bridge 502, the overall layout of the bridge 502 is provided with a correspondingly larger thickness to comply with the stability requirements of the surgical staple 502.

This embodiment provides an advantageous alternative since corresponding engagement structures of an external instrument may have a complementary shape, i.e., elongate convex protrusions. No further contact with the top surface 521 of the bridge 502 is necessary to maintain the expanded state of the surgical staple. This embodiment thus is particularly useful, when pliers or the like are used as the external instrument to hold and implant the expanded staple.

Several modifications may be made with regard to the above embodiments.

For example, in the above embodiments, the cross section of the bridge and the legs was described to be rectangular. Alternatively, the cross section may by circular, oval or polygonal, and may include rounded or chamfered edges.

In the above embodiments, the arc-shape of the bridge was described to be continuous and smooth. However, it is also possible that only portions of the bridge are curved or bended. A tenth embodiment of a surgical staple 901 is illustrated in Figs 18a - 18d. The bridge 902 includes two bending portions 909 which connect straight portions of the bridge 902. The bending portions 909 are provided adjacent a centre portion of the bridge 902, but not at the end sections 29, 29'. The surgical staple has extensions 5, 5' as projections forming engagement portions 951, 952 such that the same advantages and effects as with regard to the first embodiment of a surgical staple 1 may be achieved hereby.

Moreover, in the above embodiments, Nitinol was described as the material from which the surgical is made, or is at least partially made. Alternatively, any suitable shape memory material may be employed. Also, non-shape memory materials are encompassed by the invention. Further examples are biocompatible materials including stainless steel, titanium, beta-titanium alloys including molybdenum, vanadium, niobium, tantalum, zirconium, manganese, iron, chromium, cobalt, nickel, and copper as elements in addition to titanium. Titanium alloys provide excellent formability and reliability. Also magnesium based materials are conceivable.

In the above embodiments, the staple is described to include a bridge and two legs spanning up within one plane. However, more complex three-dimensional structures are encompassed as well. Moreover, more than two legs may be present.

In the above embodiments, the bridge of the staple is described to attain a straight flat shape when the staple is expanded. However, other shapes may also be attained in the expanded state, which is defined by the legs being arranged in parallel and ready for insertion.

In the above third example for example, a surgical staple 301 including engagement portions 351, 352 formed as projections extending along the outer side wall surfaces 322, 323 is described, wherein the projections are formed such as to extend above a plane defined by the bottom surface 324 of the bridge 302. In other words, the engagement portions 351, 352 are entirely distant from the bottom surface 324 in the third as well as in the other embodiments.

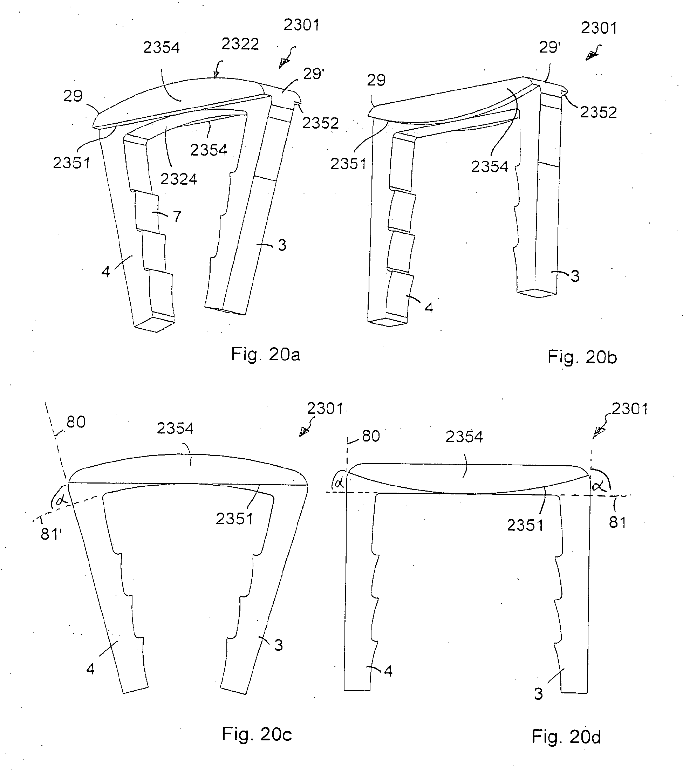

However, in another (eleventh) example of the surgical staple shown in Figs. 20a - 20d, a surgical staple 2301 has a bridge 2302 including a bottom surface 2324 and an engagement portion 2351, which approach each other in a centre portion of the bridge 2302. A bottom surface of the engagement portion 2351, 2352 thereby reveals a straight shape, when the staple 2301 is in the relaxed state as seen in Figs. 20a and 20c, whereas the same surface has a convex shape when the staple 2301 is in the expanded state as shown in Figs. 20b and 20d.

Nevertheless, as explained with respect to the example of Fig. 12b for example, the relevant parts of the projections, which extend along the outer side wall surfaces of respective bridges and which are configured for engagement by an external tool for expansion of the staple, are ideally provided at or adjacent the end portions 29, 29' of the surgical staple for the reasons of allowing sufficient torque for the expansion. In this regard, even in this example, a requirement that the engagement portions 2351, 2352 extend at least partially above a plane defined by a bottom surface 2324 of the bridge 2302, is fulfilled, such as to achieve safe placement of the staple 2301 in the bone. As a consequence of the convex arc-shape of the bottom surface of the projection 2354, the bottom surface is inclined with respect to the legs as well as with respect to plane 81 defined by the bottom wall surface 2324 of the bridge 2302, similar to the first embodiment.

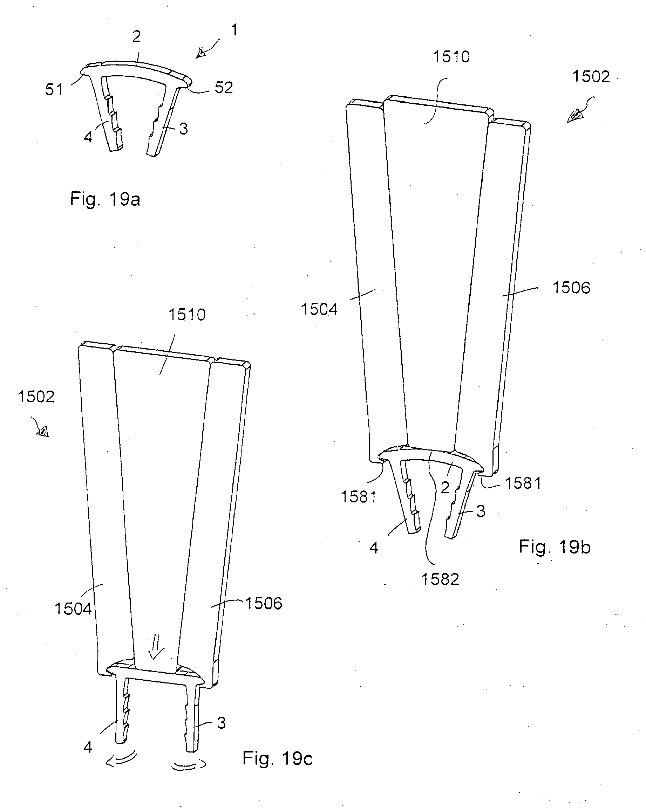

In the above embodiments of an instrument, plier instruments are described to form a cavity for receiving and holding the bridge of a staple by means of the shape of respective tip portions provided at arms or handles thereof. However, it also possible that further parts contribute to the shape of the cavity as explained with regard to a fifth embodiment of an instrument 1502 explained with respect to method steps indicated in Figs. 19a - 19c. Fig. 19a shows a step of providing the surgical staple 1 of the first embodiment having engagement portions 51, 52, formed as extensions 5, 5' of the bridge 2, see also Figs. 1a - 1d.

The plier instrument 1502 has handles or arms 1504, 1506, with tip portions similar to those described with respect to the fourth and fifth embodiments of plier instruments 1302, 1402. I.e., engagement structures 1581 are provided to engage the engagement portions 51, 52 of the bridge 2 as shown with regard to the second step illustrated in Fig. 19b.

However, a pressing member 1510 is further provided between arms or handles 1504, 1506, which has a tapered profile, such that when advanced towards the top surface 24 of the bridge 2, the arms or handles are slightly displaced away from each other. When a pressing portion 1582 provided at a front end of the pressing member 1510 abuts on the top surface 24, the bridge 2 is bent and a length between the end sections 29, 29' or extensions 5, 5' along a horizontal direction the figures increases.

However, as shown in the third step of expanding the staple 1 in Fig. 19c, the taper of pressing member 1510 allows the arms to further separate wherein a cavity is formed having the complementary shape providing the form-fit connection to the bridge 2.

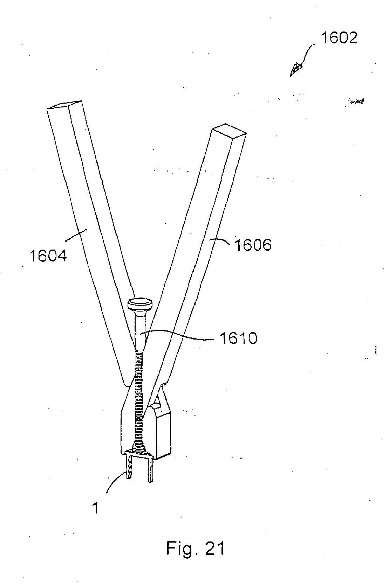

A sixth embodiment of an instrument is shown with respect to Figs. 21 - 22c. The instrument is a plier instrument 1602 having arms or handles 1604, 1606, which are rotationally coupled to each other to yield a plier. The handles 1604, 1606 include tip portions in which a cavity 1696 is formed in the manner described above with respect to the first to fifth embodiments of an instrument. The cavity 1696 includes engagement structures 1681 projection at respective ends of the cavity 1696, wherein the engagement structures 1681 are formed to engage engagement portions 51, 52 of the surgical staple according to the first embodiment.

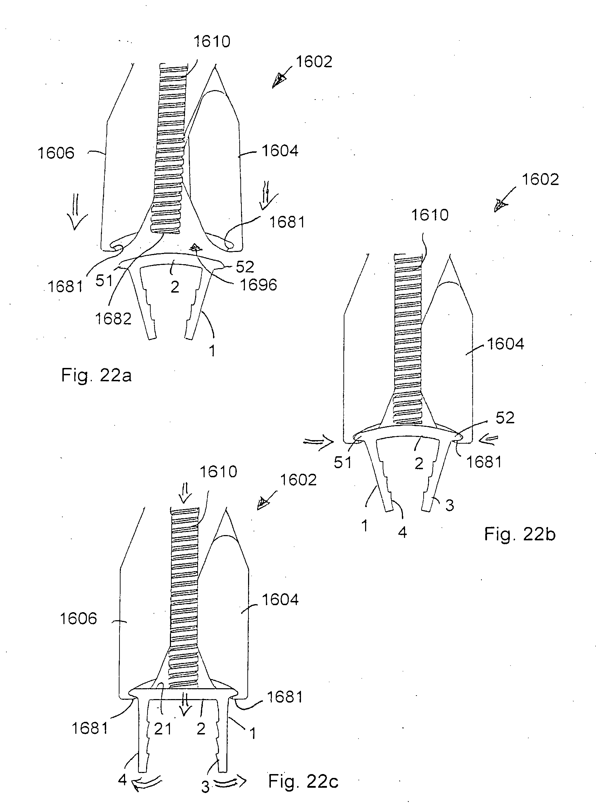

As in the fifth embodiment, a pressing portion 1682 is provided in the cavity as a surface of a separate part, which herein is a surface at a tip portion of a screw member 1610. As shown in Fig. 21, a threaded bore is provided in one or both of the handles 1604, 1606 to receive the screw member 1610 therein. Figs. 22a-22c reveal the process of the receiving and expanding the staple 1. Firstly (Fig. 22a), the handles are expanded to receive the bridge 2 of the staple including the extensions 5, 5' or engagement portions 51, 52, respectively, in the cavity 1696, which is provided by recesses formed in the tip portions of the handles 1604, 1606, by the engagement structures 1681, and by the pressing portion 1682 of screw member 1610. The screw member 1610 is in this relaxed state of the bridge 2 of staple 1 as shown in Fig. 22b in an appropriately retracted position. Next, the screw member 1610 is actuated or screwed-in to advance the pressing portion 1682 towards the top surface 21 of the bridge 2, which is indicated by the arrows in Fig,. 22c. As a consequence, the bridge 2 is bent into a straight shape whereby the legs move away from each other thereby attaining a parallel orientation and the staple 1 is finally expanded. In a next step (not shown), the staple may be applied to a bone.

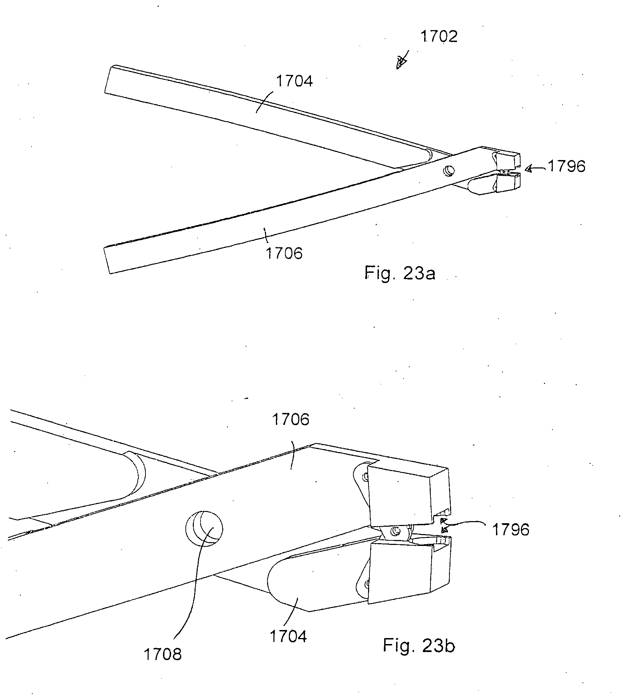

A seventh embodiment of an instrument is shown with respect to Figs. 23a - 24c. The instrument is a plier instrument 1702 having arms or handles 1704, 1706, which are rotationally coupled to each other to yield a plier. The handles 1704, 1706 include tip portions in which a cavity 1796 is formed in the manner described above with respect to the first to sixth embodiments of an instrument.

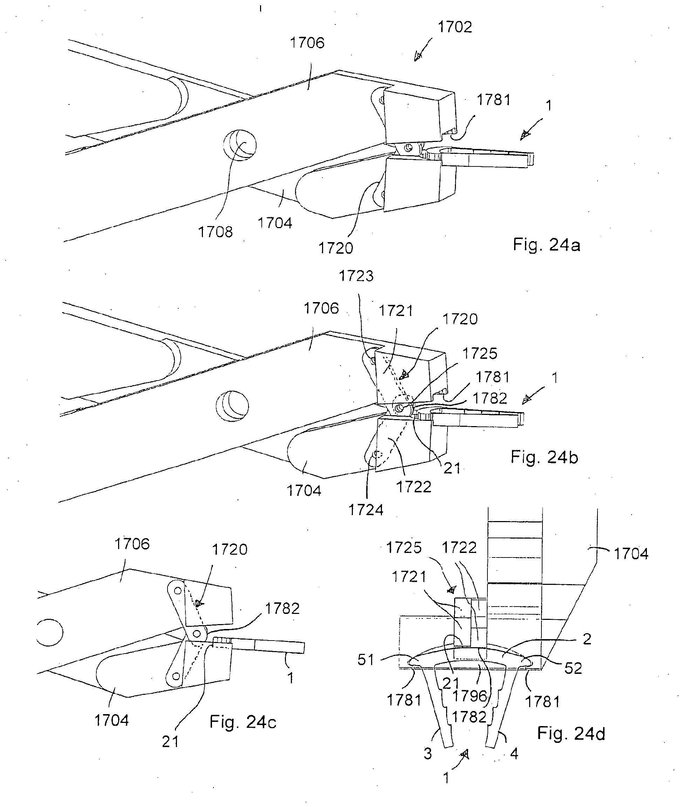

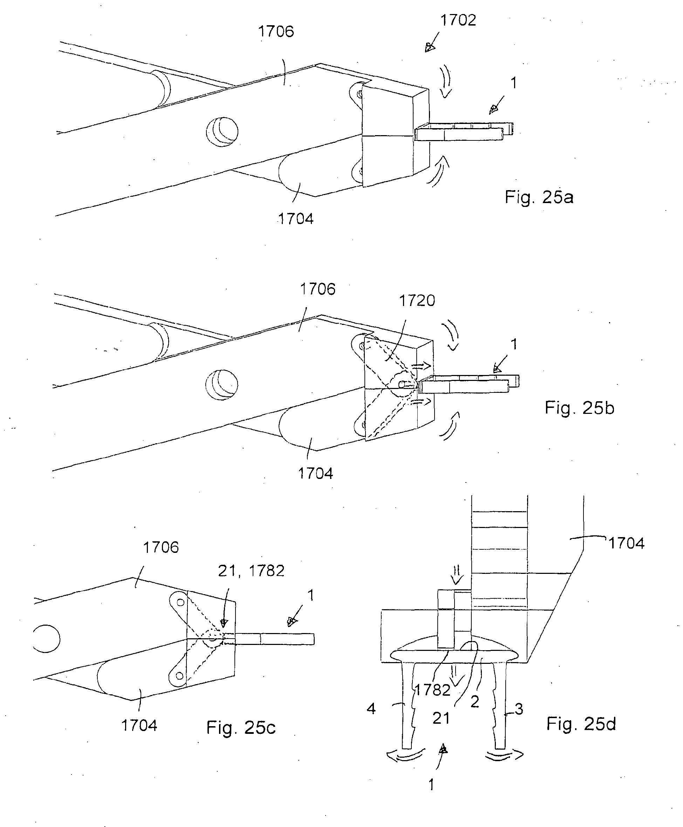

However, unlike the previous embodiments of plier instruments, the seventh embodiment provides for a combined movement of engagement structures 1781 and pressing portion 1782, which contribute in forming the cavity 1796 when expanding the surgical staple 1. The cavity 1796 extends parallel to a rotation axis of the handles (see rotational shaft hole 1708 in Fig. 23a) and not perpendicular thereto as in the previous embodiments. Hence, when opening the handles 1704, 1706, the cavity 1796 also opens and the staple 1 may be inserted into the cavity 1796 (see Figs. 24a-24d). In this specific embodiment, both tip portions include almost identical complementary shapes of the cavity. When the handles are closed, the cavity 1796 is also closed (see Figs. 25a-25d), the bridge 2 of the surgical staple 1 is safely received in the cavity 1796 in a form-fit manner.

Expansion of the surgical staple 1 is effected by means of a toggle lever 1720 which is best seen in the partially transparent views of the instrument in Figs. 24b and 25b. The toggle lever has two lever arms 1721, 1722, which are rotationally coupled at one end (via joints 1723, 1724) thereof to each of the handles 1704, 1706, more specifically to the respective tip portions of the handles. The other ends of both lever arms are rotationally coupled to each other (joint 1725). Opening and closing the handles 1704, 1706 of the plier instrument 1702 thus yields an advancement and retraction of the mutually coupled ends to and from the cavity. The mutually coupled ends of lever arms 1721, 1722 thereby form a pressing portion 1782 shaped and positioned to abut on a centre portion of the bridge 2 when advanced into the cavity 1796 in conjunction with a closing movement of the handles 1704, 1706.

## Claims

1. A surgical staple (1, 101, 901) for compressing bones or bone fragments, including
a first leg (3) for anchoring in a first bone or bone fragment;
a second leg (4) for anchoring in a second bone or bone fragment;
a bridge (2, 902) connecting the first leg (3) and the second leg (4), the bridge having a first end section (29) and a second end section (29') opposite the first end section (29), wherein the first leg (3) is connected to the first end section (29) and the second leg (4) is connected to the second end section (29');
wherein the bridge comprises two engagement portions (51, 52, 951, 952) for engagement by an external instrument provided to bend the bridge in order to transfer the surgical staple from a relaxed state to an expanded state,
wherein each of the engagement portions is formed at the first and second end sections (29, 29'), respectively, as an extension (5, 5') integrally formed with the bridge (2) and extending beyond a position where the first or second leg (3, 4, 103, 104) is connected to the respective first or second end section (29, 29') of the bridge (2),
wherein, in a relaxed state (1101) of the surgical staple, the bridge is arc-shaped in at least a portion thereof in a plane defined by the bridge and the first and second legs, and has a straight shape in an expanded state (1102) of the surgical staple, when engaged by the instrument and bent,
wherein in the expanded state (1102) of the surgical staple, a bottom side surface (24, 224, 324, 424, 2324) of the bridge defines a plane (81), and
the engagement portions are entirely located above said plane (81) and away from the legs (3, 4).

2. The surgical staple (1, 101, 901) of claim 1, wherein
in the relaxed state (1101) of the surgical staple, the first leg (3; 103) and the second leg (4; 104) extend from the bridge towards each other such that respective leg axes (80) intersect each other at an angle (&), wherein
the angle (ϑ) preferably amounts between 20 and 40 degrees, more preferably between 25 and 35 degrees and most preferable at about 30 degrees.

3. The surgical staple (1, 101, 901) of one of claims 1 to 2, wherein
in the expanded state (1102) of the surgical staple, the legs (3, 4; 103, 104) extend parallel to each other.

4. The surgical staple (1, 101, 901) of one of claims 1 to 3, wherein
the legs extend from the bridge at a same angle, preferably at 90 degrees, in the relaxed state and in the expanded state.

5. The surgical staple (1, 101, 901) of one of claims 1 to 4, wherein
the bridge, the first leg and the second leg are integrally formed, and/or
the bridge, the first leg and the second leg are defined in a common single plane in the relaxed state.

6. The surgical staple (1, 101, 901) of one of claims 1 to 5, wherein
the surgical staple includes a shape memory material exhibiting superelastic properties, preferably nitinol.

7. The surgical staple (1, 101, 901) of one of claims 1 to 6, wherein
in the expanded state, a distribution of compression forces is exerted along the length of one of the legs (3, 4) in a direction parallel to the bridge and perpendicular to the leg (3) towards the other leg (4), wherein a maximum of forces is exerted in a middle third section or in a bottom third section away from the bridge as measured along the length of the leg (3).

8. Assembly comprising the surgical staple according to one of the preceding claims and an instrument (1090, 1302, 1502, 1602, 1702) for holding the surgical staple, the instrument including:
a cavity (1096, 1696, 1796) shaped to hold and receive the bridge of the surgical staple in a form-fit manner in an expanded state (1102) thereof, wherein the cavity is provided with
two engagement structures (1081, 1381, 1581, 1681, 1781) complementary in shape to and cooperating with the respective two engagement portions provided at the bridge, and with
a pressing portion (1084, 1382, 1582, 1682, 1782) arranged to contact and exert a pressing force on a centre portion of the bridge when the bridge is received and held in the cavity such as to maintain a bent state of the bridge.

9. The assembly of claim 8, wherein the engagement structures (1081, 1381, 1581, 1681, 1781) are provided at end sections of the cavity (1096, 1696, 1796) opposite with respect to each other.

## Patentansprüche

1. Eine chirurgische Klammer (1, 101, 901) zum Komprimieren von Knochen oder Knochenfragmenten, umfassend
einen ersten Schenkel (3) zum Verankern in einem ersten Knochen oder Knochenfragment;
einen zweiten Schenkel (4) zum Verankern in einem zweiten Knochen oder Knochenfragment;
eine Brücke (2,902), die den ersten Schenkel (3) und den zweiten Schenkel (4) miteinander verbindet, wobei die Brücke einen ersten Endabschnitt (29) und einen zweiten Endabschnitt (29') gegenüber dem ersten Endabschnitt (29) besitzt, wobei der erste Schenkel (3) mit dem ersten Endabschnitt (29) verbunden ist und der zweite Schenkel (4) mit dem zweiten Endabschnitt (29') verbunden ist;
wobei die Brücke zwei Eingriffsabschnitte (51, 52, 951, 952) zum Eingriff durch ein externes Instrument besitzt, das dazu vorgesehen ist, die Brücke zu biegen, um die chirurgische Klammer von einem entspannten Zustand in einen expandierten Zustand zu transferieren,
wobei die Eingriffsabschnitte jeweils entsprechend an den ersten und zweiten Endabschnitten (29,29') als eine Verlängerung (5,5') ausgebildet sind, die mit der Brücke (2) integral ausgebildet ist und sich über eine Position hinaus erstreckt, an welcher der erste oder zweite Schenkel (3, 4, 103, 104) mit dem entsprechenden ersten oder zweiten Endabschnitt (29 und 29') der Brücke (2) verbunden ist,
wobei die Brücke in einem entspannten Zustand (1101) der chirurgischen Klammer in zumindest einem Abschnitt derselben in einer Ebene gekrümmt ist, die durch die Brücke und die ersten und zweiten Schenkel festgelegt ist, und in einem expandierten Zustand (1102) der chirurgischen Klammer eine geradlinige Form besitzt, wenn sie durch das Instrument eingegriffen und gebogen wird,
wobei eine Unterseitenoberfläche (24, 224, 324, 424, 2324) der Brücke in dem expandierten Zustand (1102) der chirurgischen Klammer eine Ebene (81) festlegt, und
die Eingriffsabschnitte vollständig oberhalb der Ebene (81) und weg von den Schenkeln (3, 4) angeordnet sind.

2. Die chirurgische Klammer (1, 101, 901) gemäß Anspruch 1, wobei
sich der erste Schenkel (3; 103) und der zweite Schenkel (4; 104) in dem entspannten Zustand (1101) der chirurgischen Klammer von der Brücke in Richtung zueinander erstrecken, sodass sich die entsprechenden Schenkelachsen (80) mit einem eingeschlossenen Winkel (ϑ) schneiden, wobei
der Winkel (ϑ) vorzugsweise zwischen 20 und 40° beträgt, weiter vorzugsweise zwischen 25 und 35°, und am meisten bevorzugt bei ungefähr 30°.

3. Die chirurgische Klammer (1, 101, 901) gemäß einem der Ansprüche 1 oder 2, wobei
sich die Schenkel (3, 4; 103, 104) in dem expandierten Zustand (1102) der chirurgischen Klammer parallel zueinander erstrecken.

4. Die chirurgische Klammer (1, 101, 901) gemäß einem der Ansprüche 1 bis 3, wobei
sich die Schenkel von der Brücke in einem gleichen Winkel, vorzugsweise 90°, in dem entspannten Zustand und in dem expandierten Zustand erstrecken.

5. Die chirurgische Klammer (1, 101, 901) gemäß einem der Ansprüche 1 bis 4, wobei
die Brücke, der erste Schenkel und der zweite Schenkel integral miteinander ausgebildet sind, und/oder
die Brücke, der erste Schenkel und der zweite Schenkel in dem entspannten Zustand in einer gemeinsamen einzigen Ebene festgelegt sind.

6. Die chirurgische Klammer (1, 101, 901) gemäß einem der Ansprüche 1 bis 5, wobei
die chirurgische Klammer ein Form Gedächtnismaterial aufweist, dass superelastische Eigenschaften aufweist, vorzugsweise Nitinol.

7. Die chirurgische Klammer (1, 101, 901) gemäß einem der Ansprüche 1 bis 6, wobei
in dem expandierten Zustand eine Verteilung von Kompressionskräften entlang der Länge eines der Schenkel (3, 4) in einer Richtung parallel zu der Brücke und senkrecht zu dem Schenkel (3) in Richtung auf den anderen Schenkel (4) ausgeübt wird, wobei ein Maximalwert der Kräfte in einem mittleren Drittel-Abschnitt oder in einem unteren Drittel-Abschnitt weg von der Brücke ausgeübt wird, wenn dies entlang der Länge des Schenkels (3) gemessen wird.

8. Anordnung umfassend die chirurgische Klammer gemäß einem der vorhergehenden Ansprüche und ein Instrument (1090, 1302, 1502, 1602, 1702) zum Halten der chirurgischen Klammer, wobei das Instrument aufweist:
eine Kavität (1096, 1696, 1796), deren Form so ausgestaltet ist, dass sie die Brücke der chirurgischen Klammer in einem expandierten Zustand (1102) derselben in formschlüssiger Weise halten und aufnehmen kann, wobei die Kavität versehen ist mit:
zwei Eingriffsstrukturen (1081, 1381, 1581, 1681, 1781), deren Form komplementär ist zu den entsprechenden zwei Eingriffsabschnitten, die an der Brücke vorgesehen sind, und mit diesem zusammenwirken, und mit
einem Druckabschnitt (1084, 1382, 1582, 1682, 1782), der so eingerichtet ist, dass er einen zentralen Abschnitt der Brücke kontaktiert und auf diesen eine Druckkraft ausübt, wenn die Brücke in der Kavität aufgenommen und gehalten ist, um somit einen gebogenen Zustand der Brücke aufrechtzuerhalten.

9. Die Anordnung gemäß Anspruch 8, wobei die Eingriffsstrukturen (1081, 1381, 1581, 1681, 1781) an Endabschnitten der Kavität (1096, 1696, 1796) einander gegenüberliegend vorgesehen sind.

## Revendications

1. Agrafe chirurgicale (1, 101, 901) servant à la compression d'os ou de fragments osseux, comprenant
une première patte (3) servant à assujettir un premier os ou fragment osseux ;
une seconde patte (4) servant à assujettir un second os ou fragment osseux;
une couronne (2, 902) reliant la première patte (3) et la seconde patte (4), la couronne comportant une première section d'extrémité (29) et une seconde section d'extrémité (29') opposée à la première section d'extrémité (29),
la première patte (3) étant reliée à la première section d'extrémité (29) et la seconde patte (4) étant reliée à la seconde section d'extrémité (29');
la couronne comprenant deux parties de mise en prise (51, 52, 951, 952) servant à la mise en prise d'un instrument extérieur prévu pour déformer la couronne afin de transférer l'agrafe chirurgicale d'un état relaxé à un état élargi,
chacune des parties de mise en prise étant réalisée respectivement au niveau des première et seconde sections d'extrémité (29, 29'), sous la forme d'un prolongement (5, 5') formé d'un seul tenant avec la couronne (2) et s'étendant au-delà d'un emplacement au niveau duquel la première ou la seconde patte (3, 4, 103, 104) est reliée à la première ou la seconde section d'extrémité (29, 29') respective de la couronne (2),
la couronne, dans un état relaxé (1101) de l'agrafe chirurgicale, étant de forme arquée dans au moins une partie de celle-ci dans un dans un plan défini par la couronne et les première et seconde pattes, et présentant une forme droite dans un état élargi (1102) de l'agrafe chirurgicale, lorsque l'instrument est mis en prise avec celle-ci et la déforme,
une face de côté inférieur (24, 224, 324, 424, 2324) de la couronne définissant un plan (81) dans l'état élargi (1102) de l'agrafe chirurgicale, et
les parties de mise en prise sont situées entièrement au-dessus dudit plan (81) et à l'écart des pattes (3, 4).

2. Agrafe chirurgicale (1, 101, 901) selon la revendication 1, dans laquelle
dans l'état relaxé (1101) de l'agrafe chirurgicale, la première patte (3; 103) et la seconde patte (4; 104) s'étendent à partir de la couronne l'une en direction de l'autre de telle sorte que des axes de patte (80) respectifs se croisent mutuellement à un angle (ϑ),
l'angle (ϑ) valant de préférence entre 20 et 40 degrés, plus préférablement entre 25 et 35 degrés et le plus préférablement environ 30 degrés.

3. Agrafe chirurgicale (1, 101, 901) selon l'une des revendications 1 et 2, dans laquelle
dans l'état élargi (1102) de l'agrafe chirurgicale, les pattes (3, 4; 103, 104) s'étendent parallèlement l'une à l'autre.

4. Agrafe chirurgicale (1, 101, 901) selon l'une des revendications 1 à 3, dans laquelle
les pattes s'étendent à partir de la couronne au même angle, de préférence à 90 degrés, dans l'état relaxé et dans l'état élargi.

5. Agrafe chirurgicale (1, 101, 901) selon l'une des revendications 1 à 4, dans laquelle
la couronne, la première patte et la seconde patte sont formées d'un seul tenant, et/ou
la couronne, la première patte et la seconde patte sont définies dans un seul plan commun dans l'état relaxé.

6. Agrafe chirurgicale (1, 101, 901) selon l'une des revendications 1 à 5, dans laquelle
l'agrafe chirurgicale comprend un matériau à mémoire de forme possédant des propriétés superélastiques, de préférence le nitinol.

7. Agrafe chirurgicale (1, 101, 901) selon l'une des revendications 1 à 6, dans laquelle
dans l'état élargi, une distribution des forces de compression est exercée le long de la longueur de l'une des pattes (3, 4) dans une direction parallèle à la couronne et perpendiculaire à la patte (3) en direction de l'autre patte (4), un maximum de forces étant exercé dans un tiers médian ou dans un tiers inférieur à l'écart de la couronne en mesurant le long de la longueur de la patte (3).

8. Ensemble comprenant l'agrafe chirurgicale selon l'une des revendications précédentes et un instrument (1090, 1302, 1502, 1602, 1702) servant à maintenir l'agrafe chirurgicale, l'instrument comprenant :
une cavité (1096, 1696, 1796) façonnée pour maintenir et recevoir la couronne de l'agrafe chirurgicale selon un accouplement géométrique dans un état élargi (1102) de celle-ci, la cavité étant pourvue de
deux structures de mise en prise (1081, 1381, 1581, 1681, 1781) présentant une forme complémentaire de celle des deux parties de mise en prise respectives formées au niveau de la couronne et coopérant avec celles-ci, et
d'une partie de pression (1084, 1382, 1582, 1682, 1782) conçue pour venir en contact avec une partie centrale de la couronne et exercer une pression sur celle-ci lorsque la couronne est reçue et maintenue dans la cavité de façon à maintenir un état déformé de la couronne.

9. Ensemble selon la revendication 8, dans lequel les structures de mise en prise (1081, 1381, 1581, 1681, 1781) sont formées au niveau de sections d'extrémité de la cavité (1096, 1696, 1796) opposées l'une à l'autre.
